# EUROPEAN PATENT SPECIFICATION

(11) **EP 4 028 126 B1**
(45) Date of publication and mention of the grant of the patent: **26.02.2025**
(21) Application number: 20781171.2
(22) Date of filing: 11.09.2020
(51) Int. Cl.: A61K 38/12, A61P 21/00, A61P 21/04, G01N 33/564, G01N 33/68

(54) **TREATMENT OF A NEUROLOGICAL DISEASE WITH COMPLEMENT INHIBITORS**
BEHANDLUNG EINER NEUROLOGISCHEN ERKRANKUNG MIT KOMPLEMENTINHIBITOREN
TRAITEMENT D'UNE MALADIE NEUROLOGIQUE AVEC DES INHIBITEURS DU COMPLÉMENT

(30) Priority: 12.09.2019 US 201962899488 P; 04.02.2020 US 202062969742 P; 12.03.2020 US 202062988587 P; 08.05.2020 US 202063021742 P
(43) Date of publication of application: 20.07.2022
(73) Proprietor: RA Pharmaceuticals, Inc., Cambridge, MA 02140 (US)
(72) Inventor: READ, Simon J., Lexington, Massachusetts 02421 (US); RICARDO, Alonso, Winchester, Massachusetts 01890 (US); SAYEGH, Camil, Belmont, Massachusetts 02478 (US); XU, Xiangyang, Auburndale, Massachusetts 02466 (US); TANG, Yalan, Cambridge, Massachusetts 02140 (US); TANG, Guo-Qing, Acton, Massachusetts 01720 (US)
(74) Representative: Hoffmann Eitle
(86) International application number: PCT/US2020/050422
(87) International publication number: WO 2021/050885

(56) References cited:
- WO-A2-2020/185541
- US-A1- 2017 137 468
- ANONYMOUS: "HEALEY ALS Platform Trial - Regimen A Zilucoplan", 3 August 2020 (2020-08-03), XP055750035, Retrieved from the Internet <URL:https://clinicaltrials.gov/ct2/history/NCT04436497?V_2=View#StudyPageTop> [retrieved on 20201112]
- JOHN D LEE ET AL: "Pharmacological inhibition of complement C5a-C5a 1 receptor signalling ameliorates disease pathology in the hSOD1 G93A mouse model of amyotrophic lateral sclerosis : C5a 1 receptor antagonism is protective in ALS mice", BRITISH JOURNAL OF PHARMACOLOGY, vol. 174, no. 8, 3 March 2017 (2017-03-03), UK, pages 689 - 699, XP055749791, ISSN: 0007-1188, DOI: 10.1111/bph.13730
- BRIAN PARK: "Zilucoplan for Myasthenia Gravis Gets Orphan Drug Designation - Neurology Advisor", 9 September 2019 (2019-09-09), XP055750534, Retrieved from the Internet <URL:https://www.neurologyadvisor.com/topics/neuromuscular-disorders/zilucoplan-for-myasthenia-gravis-treatment-gets-orphan-drug-designation/> [retrieved on 20201113]
- ANONYMOUS: "NCT04436497: HEALEY ALS Platform Trial - Regimen A Zilucoplan", 17 June 2020 (2020-06-17), XP055750522, Retrieved from the Internet <URL:https://clinicaltrials.gov/ct2/history/NCT04436497?V_1=View#StudyPageTop> [retrieved on 20201113]
- SVITLANA GARBUZOVA-DAVIS ET AL: "Blood-CNS Barrier Impairment in ALS patients versus an animal model", FRONTIERS IN CELLULAR NEUROSCIENCE, vol. 8, 1 January 2014 (2014-01-01), XP055750514, DOI: 10.3389/fncel.2014.00021

## Description

### CROSS REFERENCE TO RELATED APPLICATIONS

This application claims priority to United States Provisional Application Number 62/899,488 filed on September 12, 2019 entitled NEUROLOGICAL DISEASE TREATMENT WITH COMPLEMENT INHIBITORS, United States Provisional Application Number 62/969,742 filed on February 4, 2020 entitled NEUROLOGICAL DISEASE TREATMENT WITH COMPLEMENT INHIBITORS, United States Provisional Application Number 62/988,587 filed on March 12, 2020 entitled NEUROLOGICAL DISEASE TREATMENT WITH COMPLEMENT INHIBITORS, and United States Provisional Application Number 63/021,742 filed on May 8, 2020 entitled NEUROLOGICAL DISEASE TREATMENT WITH COMPLEMENT INHIBITORS.

### SEQUENCE LISTING

The present application is being filed along with a Sequence Listing in electronic format. The Sequence Listing file, entitled 2011_1058PCT_SL.txt, was created on September 11, 2020 and is 1,231 bytes in size.

### BACKGROUND

The vertebrate immune response is comprised of adaptive and innate immune components. While the adaptive immune response is selective for particular pathogens and is slow to respond, components of the innate immune response recognize a broad range of pathogens and respond rapidly upon infection. One such component of the innate immune response is the complement system.

The complement system includes about 20 circulating complement component proteins, synthesized primarily by the liver. Components of this particular immune response were first termed "complement" due to the observation that they complemented the antibody response in the destruction of bacteria. These proteins remain in an inactive form prior to activation in response to infection. Activation occurs by way of a pathway of proteolytic cleavage initiated by pathogen recognition and leading to pathogen destruction. Three such pathways are known in the complement system and are referred to as the classical pathway, the lectin pathway, and the alternative pathway. The classical pathway is activated when an IgG or IgM molecule binds to the surface of a pathogen. The lectin pathway is initiated by the mannan-binding lectin protein recognizing the sugar residues of a bacterial cell wall. The alternative pathway remains active at low levels in the absence of any specific stimuli. While all three pathways differ with regard to initiating events, all three pathways converge with the cleavage of complement component C3. C3 is cleaved into two products termed C3a and C3b. Of these, C3b becomes covalently linked to the pathogen surface while C3a acts as a diffusible signal to promote inflammation and recruit circulating immune cells. Surface-associated C3b forms a complex with other components to initiate a cascade of reactions among the later components of the complement system. Due to the requirement for surface attachment, complement activity remains localized and minimizes destruction to non-target cells.

Pathogen-associated C3b facilitates pathogen destruction in two ways. In one pathway, C3b is recognized directly by phagocytic cells and leads to engulfment of the pathogen. In the second pathway, pathogen-associated C3b initiates the formation of the membrane attack complex (MAC). In the first step, C3b complexes with other complement components to form the C5-convertase complex. Depending on the initial complement activation pathway, the components of this complex may differ. C5-convertase formed as the result of the classical complement pathway comprises C4b and C2a in addition to C3b. When formed by the alternative pathway, C5-convertase comprises two subunits of C3b as well as one Bb component.

Complement component C5 is cleaved by either C5-convertase complex into C5a and C5b. C5a, much like C3a, diffuses into the circulation and promotes inflammation, acting as a chemoattractant for inflammatory cells. C5b remains attached to the cell surface where it triggers the formation of the MAC through interactions with C6, C7, C8 and C9. The MAC is a hydrophilic pore that spans the membrane and promotes the free flow of fluid into and out of the cell, thereby destroying it.

An important component of all immune activity is the ability of the immune system to distinguish between self and non-self cells. Pathology arises when the immune system is unable to make this distinction. In the case of the complement system, vertebrate cells express proteins that protect them from the effects of the complement cascade. This ensures that targets of the complement system are limited to pathogenic cells. Many complement-related disorders and diseases are associated with inflammation that leads to or arises from abnormal destruction of self cells by the complement cascade.

Amyotrophic lateral sclerosis (ALS) is a progressive disease causing bodily muscle weakness. Disease progression varies between individuals with three-year median survival with onset of symptoms and 80% patient death within five years. Although a specific cause of disease is unknown, complement-related immune dysregulation is thought to be a contributing factor. Complement components are synthesized by cells of the central nervous system, including neurons, astrocytes, oligodendrocytes, and microglia. Further, biomarkers of classical complement activation pathway (C1q and C4) have been identified in association ALS serum, cerebrospinal fluid, motor cortex and spinal cord parenchyma, and skeletal muscle tissue.

Lee et al., 2017. Br J Pharmacol. 174(8):689-699 discloses the selective and orally active C5a₁ receptor inhibitor PMX205. According to Lee *et al.,* PMX205 can improve hindlimb grip strength, slower disease progression and extend survival in hSOD1^{G93A} mice when administered before disease onset.

There remains a need in the field for compounds and therapeutic methods for addressing complement-related diseases and disorders, including those affecting the nervous system, such as ALS. The present disclosure meets this need by providing related compounds and methods of treatment.

### SUMMARY

The invention is defined by the appended claims. Any subject-matter falling outside of the scope of the claims is provided for information purposes, only. The references to methods of treatment in this description are to be interpreted as references to the compounds, pharmaceutical compositions and medicaments of the present invention for use in a method for treatment of the human or animal body.

The present invention provides zilucoplan for use in a method of treating ALS in a subject, the method comprising administering zilucoplan to the subject. Zilucoplan is a C5 complement inhibitor. Prior to treatment, subject ALS disease severity may be elevated and/or may be increasing at a specific rate. Complement inhibitor treatment may stabilize or reduce subject ALS disease severity and/or reduce the specific rate at which subject ALS disease severity is increasing. Change in subject ALS disease severity may be measured by ALS Functional Rating Scale-Revised (ALSFRS-R). Prior to treatment, subject respiratory function may be decreased and/or declining at a specific rate. Complement inhibitor treatment may stabilize or improve subject respiratory function and/or reduce the specific rate at which subject respiratory function is declining. Change in subject respiratory function may be assessed by slow vital capacity (SVC). Prior to treatment, subject muscle strength may be decreased and/or declining at a specific rate. Complement inhibitor treatment may stabilize or improve subject muscle strength and/or reduce the specific rate at which subject muscle strength is declining. Change in subject muscle strength may be measured by hand held dynamometry (HHD). Complement inhibitor treatment may stabilize or improve subject speech capability and/or stabilize or improve a rate of subject speech capability decline. Subject speech capability may include one or more of articulatory precision, speaking rate, percent of speech signal voiced, phonation length, consonant length, and nasality. Complement inhibitor treatment may stabilize or improve subject ALS biomarker profile and/or rate of subject ALS biomarker profile change. The subject ALS biomarker profile may include a biomarker selected from one or more of neurofilament heavy chain (pNfH), neurofilament light chain (NfL), C5, and a complement activity biomarker. The complement activity biomarker may be selected from one or more of C5a, C5b-9, soluble C5b-9, C3a, C4a, Ba, Bb, Factor I, and Factor H. The complement activity biomarker may be detected in subject cerebrospinal fluid (CSF), plasma, and/or serum. The subject may have or may be suspected of having myasthenia gravis. The subject may be positive for at least one autoantibody. The at least one autoantibody may be one or more of anti-acetylcholine receptor antibody, anti-LDL receptor related protein 4, anti-agrin, and anti-muscle associated receptor tyrosine kinase. The subject may have impaired blood brain barrier function. The impaired blood brain barrier function may be assessed by determining the subject's albumin quotient. The subject may be determined to have definite ALS or probable ALS based on revised El Escorial criteria.

ALS can be diagnosed in a subject by detecting and/or quantifying complement activity associated with the subject or a biological sample from the subject and diagnosing the subject with ALS based on complement activity detected and/or quantified. Complement activity detection and/or quantification may include detecting and/or quantifying a complement activity biomarker. The complement activity biomarker may be selected from one or more of C5a, C5b-9, soluble C5b-9, C3a, C4a, Ba, Bb, Factor I, and Factor H. The complement activity biomarker may be detected and/or quantified in subject CSF, plasma, and/or serum.

ALS in a subject can be monitored by (1) detecting and/or quantifying complement activity associated with: (a) the subject at an initial time point or over an initial time period; or (b) a biological sample obtained from the subject at the initial time point or over the initial time period; and (2) detecting and/or quantifying complement activity associated with: (a) the subject at a subsequent time point or over a subsequent time period; or (b) a biological sample obtained from the subject at the subsequent time point or over the subsequent time period; and (3) monitoring ALS in the subject by assessing any change in complement activity detected and/or quantified between the initial time point or period and the subsequent time point or period. Complement activity detection and/or quantification may include detecting and/or quantifying a complement activity biomarker. The complement activity biomarker may be selected from one or more of C5a, C5b-9, soluble C5b-9, C3a, C4a, Ba, Bb, Factor I, and Factor H. The complement activity biomarker may be detected and/or quantified in subject CSF, plasma, and/or serum. The subject may be determined to have definite ALS or probable ALS based on revised El Escorial criteria.

Subjects with ALS or suspected of having ALS can be stratified into two or more groups, by detecting and/or quantifying complement activity associated with each subject or a biological sample from each subject and assigning each subject to at least one of the two or more groups based on complement activity detected and/or quantified. Complement activity detection and/or quantification may include detecting and/or quantifying a complement activity biomarker. The complement activity biomarker may be selected from one or more of C5a, C5b-9, soluble C5b-9, C3a, C4a, Ba, Bb, Factor I, and Factor H. The complement activity biomarker may be detected and/or quantified in subject CSF, plasma, and/or serum. Subjects may be assigned to a treatment group based on CSF, plasma, and/or serum complement activity biomarker levels that are elevated in comparison to CSF, plasma, and/or serum complement activity biomarker levels in subjects without ALS. The treatment group may be characterized by eligibility for zilucoplan treatment. The subject may be determined to have definite ALS or probable ALS based on revised El Escorial criteria.

The dose and/or regimen of complement inhibitor administered to a subject according to treatment methods described herein may be determined based on the level of a complement activity biomarker detected in CSF, plasma, and/or serum obtained from the subject prior to treatment. The complement activity biomarker detected in CSF, plasma, and/or serum obtained from the subject prior to treatment may be selected from one or more of C5a, C5b-9, soluble C5b-9, C3a, C4a, Ba, Bb, Factor I, and Factor H. The dose and/or regimen of complement inhibitor administered may be adjusted after one or more prior complement inhibitor administration based on the level of a complement activity biomarker detected in CSF, plasma, and/or serum obtained from the subject after one or more prior administration. The complement activity biomarker detected in CSF, plasma, and/or serum obtained from the subject after one or more prior administration may be selected from one or more of C5a, C5b-9, soluble C5b-9, C3a, C4a, Ba, Bb, Factor I, and Factor H. The complement inhibitor administered may be zilucoplan.

In some embodiments, the subject has or is suspected of having an impaired blood brain barrier. The subject may be determined to have definite ALS or probable ALS based on revised El Escorial criteria. The subject may be assessed for blood brain barrier impairment by determining the subject's albumin quotient.

Selecting a subject for treatment may include assessing the presence or level of at least one biomarker in a subject sample and selecting the subject for treatment with the complement inhibitor based on the assessment of the presence or level of the at least one biomarker in the subject sample. The at least one biomarker may include one or more of a neuroaxonal degeneration biomarker, a neuroinflammation biomarker, and a complement activity biomarker. The subject sample may include a plasma sample and/or a CSF sample. The neuroaxonal degeneration biomarker may be pNfH and/or NfL. The neuroinflammation biomarker may include monocyte chemoattractant protein-1 (MCP-1), chitotriosidase-1 (CHIT1), and/or chitinase-3-like protein 1 (YKL-40). The complement activity biomarker may include C5a, C5b-9, sC5b-9, C5b6 complex, C3a, C4a, Ba, Bb, Factor I, and/or Factor H. Levels of one or more of Nfl, pNfH, CHIT1, C5a, and sC5b-9 may be elevated in the subject sample relative to levels in a subject sample from a subject without ALS. The subject sample and the subject sample from a subject without ALS may include CSF. The subject sample may be obtained from a subject with impaired blood brain barrier function. One or more of C3a, Bb, and Ba may be elevated in the subject sample relative to levels in a subject sample from a subject without ALS. The subject sample and the subject sample from a subject without ALS may include plasma. C3a levels may be elevated in the subject sample relative to levels in a subject sample from a subject without ALS.

In some embodiments, the subject for treatment is selected according to any of the methods described herein [e.g., based on assessing the presence or level of at least one biomarker (e.g., a neuroaxonal degeneration biomarker, a neuroinflammation biomarker, and/or a complement activity biomarker).

In some embodiments, zilucoplan is comprised in a composition and the composition reduces, prevents, stabilizes, or reverses one or more effects of ALS experienced by the subject. Prior to treatment, subject ALS disease severity may be elevated and/or may be increasing at a specific rate. The treatment may stabilize or reduce subject ALS disease severity and/or reduce the specific rate at which subject ALS disease severity is increasing. Change in subject ALS disease severity may be measured by ALSFRS-R. Prior to treatment, subject respiratory function may be decreased and/or may be declining at a specific rate. The treatment with the complement inhibitor may stabilize or improve subject respiratory function and/or reduce the specific rate at which subject respiratory function is declining. Change in subject respiratory function may be assessed by SVC. Prior to treatment, subject muscle strength may be decreased and/or declining at a specific rate and treatment with the composition may stabilize or improve subject muscle strength and/or reduce the specific rate at which subject muscle strength is declining. Change in subject muscle strength may be measured by HHD. The treatment may stabilize or improve subject speech capability and/or rate of subject speech capability decline. Subject speech capability may include one or more of articulatory precision, speaking rate, percent of speech signal voiced, phonation length, consonant length, and nasality. The treatment may stabilize or improve subject ALS biomarker profile and/or rate of subject ALS biomarker profile change. The subject ALS biomarker profile may include a biomarker selected from one or more of pNfH, NfL, C5, and a complement activity biomarker. The complement activity biomarker may be selected from one or more of C5a, C5b-9, soluble C5b-9, C3a, C4a, Ba, Bb, Factor I, and Factor H. The complement activity biomarker may be detected in subject CSF, plasma, and/or serum. The dose and/or regimen of the composition may be adjusted based on the level of a complement activity biomarker detected in a CSF sample, plasma sample, and/or serum sample obtained from the subject after one or more prior composition administration. The complement activity biomarker detected in the CSF sample, plasma sample, and/or serum obtained from the subject after one or more prior composition administration may be selected from one or more of C5a, C5b-9, soluble C5b-9, C3a, C4a, Ba, Bb, Factor I, and Factor H. The subject may have or be suspected of having myasthenia gravis. The subject may be positive for at least one autoantibody. The at least one autoantibody may include one or more of anti-acetylcholine receptor antibody, anti-LDL receptor related protein 4, anti-agrin, and anti-muscle associated receptor tyrosine kinase. The subject may have impaired blood brain barrier function. The impaired blood brain barrier function may be assessed by determining the subject's albumin quotient. The subject may be determined to have definite ALS or probable ALS based on revised El Escorial criteria.

ALS in a subject can be diagnosed by detecting and/or quantifying complement activity associated with a biological sample obtained from the subject and diagnosing the subject with ALS based on complement activity detected and/or quantified. The step of detecting and/or quantifying complement activity associated with the biological sample may include detecting and/or quantifying a complement activity biomarker in the biological sample. The complement activity biomarker may be selected from one or more of C5a, C5b-9, soluble C5b-9, C3a, C4a, Ba, Bb, Factor I, and Factor H. The biological sample may include subject CSF, plasma, and/or serum.

ALS in a subject can be monitored by detecting and/or quantifying complement activity associated with a biological sample obtained from the subject at an initial time point or over an initial time period; detecting and/or quantifying complement activity associated with a biological sample obtained from the subject at a subsequent time point or over a subsequent time period; and monitoring ALS in the subject by assessing any change in complement activity detected and/or quantified between the biological sample obtained from the subject at the initial time point or over the initial time period and the biological sample obtained from the subject at the subsequent time point or over the subsequent time period. Complement activity detection and/or quantification may include detecting and/or quantifying a complement activity biomarker. The complement activity biomarker may be selected from one or more of C5a, C5b-9, soluble C5b-9, C3a, C4a, Ba, Bb, Factor I, and Factor H. The biological sample obtained from the subject at the initial time point or over the initial time period and/or the biological sample obtained from the subject at the subsequent time point or over the subsequent time period may include CSF, plasma, and/or serum. The subject may have definite ALS or probable ALS, as assessed based on revised El Escorial criteria.

Subjects with ALS or suspected of having ALS can be stratified into two or more groups by detecting and/or quantifying complement activity associated with a biological sample obtained from each subject and assigning each subject to at least one of the two or more groups based on complement activity detected and/or quantified. Complement activity detection and/or quantification may include detecting and/or quantifying a complement activity biomarker. The complement activity biomarker may be selected from one or more of C5a, C5b-9, soluble C5b-9, C3a, C4a, Ba, Bb, Factor I, and Factor H. The biological sample obtained from each subject may include CSF, plasma, and/or serum. The two or more groups may include a treatment group, wherein the subjects are assigned to the treatment group based on CSF, plasma, and/or serum complement activity biomarker levels that are elevated in comparison to CSF, plasma, and/or serum complement activity biomarker levels in subjects without ALS. The treatment group may be characterized by eligibility for zilucoplan treatment. The subject may be determined to have definite ALS or probable ALS based on revised El Escorial criteria. The dose and/or regimen of the composition may be determined based on the level of a complement activity biomarker detected in a CSF sample, plasma sample, and/or serum obtained from the subject prior to treatment. The complement activity biomarker detected in the CSF sample, plasma sample, and/or serum obtained from the subject prior to treatment may be selected from one or more of C5a, C5b-9, soluble C5b-9, C3a, C4a, Ba, Bb, Factor I, and Factor H.

In some embodiments, zilucoplan is comprised in a composition and the subject has or is suspected of having an impaired blood brain barrier, wherein the composition reduces, prevents, stabilizes, or reverses one or more effects of the complement-related indication. The subject may be determined to have definite ALS or probable ALS based on revised El Escorial criteria. The subject may be assessed for blood brain barrier impairment by determining the subject's albumin quotient.

In some embodiments, zilucoplan is comprised in a composition and the method includes assessing the presence or level of at least one biomarker in a subject sample and selecting the subject for treatment with zilucoplan based on the assessment of the presence or level of the at least one biomarker in the subject sample. The at least one biomarker may include one or more of a neuroaxonal degeneration biomarker, a neuroinflammation biomarker, and a complement activity biomarker. The subject sample may include a plasma sample and/or a CSF sample. The neuroaxonal degeneration biomarker may be pNfH and/or NfL. The neuroinflammation biomarker may include MCP-1, CHIT1, and/or YKL-40. The complement activity biomarker may include C5a,y includ C5b-9, sC5b-9, C5b6 complex, C3a, C4a, Ba, Bb, Factor I, and/or Factor H. Levels of one or more of Nfl, pNfH, CHIT1, C5a, and sC5b-9 may be elevated in the subject sample (e.g., CSF) relative to levels in a subject sample from a subject without ALS. The subject sample may be obtained from a subject with impaired blood brain barrier function. One or more of C3a, Bb, and Ba may be elevated in the subject sample (e.g., plasma) relative to levels in a subject sample from a subject without ALS. C3a levels may be elevated in the subject sample relative to levels in a subject sample from a subject without ALS.

### BRIEF FIGURE DESCRIPTION

The foregoing and other objects, features and advantages of particular embodiments of the disclosure will be apparent from the following description and illustrations in the accompanying figures.
Fig. 1 is a graph showing fold change in CSF biomarker levels from subjects with impaired blood brain barrier (as determined by CSF albumin:plasma albumin ratio). Statistical analysis was performed using Kruskal-Wallis test (*p<0.05, **p<0.01, ***p<0.001, ****p<0.0001). Whiskers indicate 5-95 percentile and "+" within boxes denotes mean.
Fig. 2 is a graph showing fold change in CSF biomarker levels from subjects with intact blood brain barrier (as determined by CSF albumin:plasma albumin ratio). Statistical analysis was performed using Kruskal-Wallis test (*p<0.05, **p<0.01, ***p<0.001, ****p<0.0001). Whiskers indicate 5-95 percentile and "+" within boxes denotes mean.
Fig. 3 is a graph showing median levels of C5a in plasma samples from healthy control subjects compared with median levels in subjects with definite or probable ALS. Error bars indicate 95% confidence interval. Significance value was determined by Mann-Whitney test with p>0.05 threshold.
Fig. 4 is a graph showing median levels of sC5b9 in plasma samples from healthy control subjects compared with median levels in subjects with definite or probable ALS. Error bars indicate 95% confidence interval. Significance value was determined by Mann-Whitney test with p>0.05 threshold.
Fig. 5 is a graph showing median levels of C3a in plasma samples from healthy control subjects compared with median levels in subjects with definite or probable ALS. Error bars indicate 95% confidence interval. Significance value was determined by Mann-Whitney test with p>0.05 threshold.
Fig. 6 is a graph showing median levels of C4a in plasma samples from healthy control subjects compared with median levels in subjects with definite or probable ALS. Error bars indicate 95% confidence interval. Significance value was determined by Mann-Whitney test with p>0.05 threshold.
Fig. 7 is a graph showing median levels of Ba in plasma samples from healthy control subjects compared with median levels in subjects with definite or probable ALS. Error bars indicate 95% confidence interval. Significance value was determined by Mann-Whitney test with p>0.05 threshold.
Fig. 8 is a graph showing median levels of Bb in plasma samples from healthy control subjects compared with median levels in subjects with definite or probable ALS. Error bars indicate 95% confidence interval. Significance value was determined by Mann-Whitney test with p>0.05 threshold.
Fig. 9 is a graph showing median levels of fH in plasma samples from healthy control subjects compared with median levels in subjects with definite or probable ALS. Error bars indicate 95% confidence interval. Significance value was determined by Mann-Whitney test with p>0.05 threshold.
Fig. 10 is a graph showing median levels of fI in plasma samples from healthy control subjects compared with median levels in subjects with definite or probable ALS. Error bars indicate 95% confidence interval. Significance value was determined by Mann-Whitney test with p>0.05 threshold.
Fig. 11 is a graph showing median levels of C5a in CSF samples from healthy control subjects compared with median levels in subjects with definite or probable ALS. Error bars indicate 95% confidence interval. Significance value was determined by Mann-Whitney test with p>0.05 threshold.
Fig. 12 is a graph showing median levels of sC5b9 in CSF samples from healthy control subjects compared with median levels in subjects with definite or probable ALS. Error bars indicate 95% confidence interval. Significance value was determined by Mann-Whitney test with p>0.05 threshold.
Fig. 13 is a graph showing median levels of C3a in CSF samples from healthy control subjects compared with median levels in subjects with definite or probable ALS. Error bars indicate 95% confidence interval. Significance value was determined by Mann-Whitney test with p>0.05 threshold.
Fig. 14 is a graph showing median levels of C4a in CSF samples from healthy control subjects compared with median levels in subjects with definite or probable ALS. Error bars indicate 95% confidence interval. Significance value was determined by Mann-Whitney test with p>0.05 threshold.
Fig. 15 is a graph showing median levels of Ba in CSF samples from healthy control subjects compared with median levels in subjects with definite or probable ALS. Error bars indicate 95% confidence interval. Significance value was determined by Mann-Whitney test with p>0.05 threshold.
Fig. 16 is a graph showing median levels of Bb in CSF samples from healthy control subjects compared with median levels in subjects with definite or probable ALS. Error bars indicate 95% confidence interval. Significance value was determined by Mann-Whitney test with p>0.05 threshold.
Fig. 17 is a graph showing median levels of fH in CSF samples from healthy control subjects compared with median levels in subjects with definite or probable ALS. Error bars indicate 95% confidence interval. Significance value was determined by Mann-Whitney test with p>0.05 threshold.
Fig. 18 is a graph showing median levels of fI in CSF samples from healthy control subjects compared with median levels in subjects with definite or probable ALS. Error bars indicate 95% confidence interval. Significance value was determined by Mann-Whitney test with p>0.05 threshold.
Fig. 19 is a graph showing albumin quotient values determined using plasma and CSF samples from healthy control subjects and subjects with definite or probable ALS. Levels associated with intact, mildly impaired, or moderately impaired blood brain barrier are indicated. Error bars indicate 95% confidence interval. Significance value was determined by Mann-Whitney test with p>0.05 threshold.
Fig. 20 is a graph showing neurofilament light chain (NfL) and phosphorylated neurofilament heavy chain (pNfH) biomarker levels in CSF from healthy control subjects and subjects with definite or probable ALS. For NfL in ALS, N=28 and for pNfH in ALS, N=17. Error bars indicate 95% confidence interval. Significance values were determined by Mann-Whitney test (2 groups) or Kruskal-Wallis test (3 groups).
Fig. 21 is a graph showing chitotriosidase-1 (CHIT1) and chitinase-3-like protein 1 (YKL-40) biomarker levels in CSF from healthy control subjects and subjects with ALS (N=17). Error bars indicate 95% confidence interval. Significance values were determined by Mann-Whitney test (2 groups) or Kruskal-Wallis test (3 groups). "ns" indicates no significant difference.

### DETAILED DESCRIPTION

### Introduction

Embodiments of the present disclosure relate to compounds and compositions for modulating complement activity and related methods of use. Complement activity protects the body from foreign pathogens but can lead to self-cell destruction with elevated activity or poor regulation. Complement modulators may be complement inhibitors, such as zilucoplan. Zilucoplan is a synthetic, macrocyclic peptide that binds complement component 5 (C5) with sub-nanomolar affinity and allosterically inhibits its cleavage into C5a and C5b upon activation of the classical, alternative, or lectin pathways (see, e.g., United States Patent Number 10,106,579).

Included herein is zilucoplan for use in a method of treating ALS by administering zilucoplan. Zilucoplan may be used to treat ALS by reducing or eliminating the harmful effects of complement-mediated self-tissue destruction associated with overactive complement. These and other embodiments of the disclosure are described in detail below.

### I. Compounds and compositions

As used herein, "complement activity" includes the activation of the complement cascade, the formation of cleavage products from a complement component such as C3 or C5, the assembly of downstream complexes following a cleavage event, or any process or event attendant to, or resulting from, the cleavage of a complement component, e.g., C3 or C5. Complement inhibitors may include C5 inhibitors that block complement activation at the level of complement component C5. C5 inhibitors may bind C5 and prevent its cleavage, by C5 convertase, into the cleavage products C5a and C5b. As used herein, "Complement component C5" or "C5" is defined as a complex which is cleaved by C5 convertase into at least the cleavage products, C5a and C5b. "C5 inhibitors," as referred to herein, include any compound or composition that inhibits the processing or cleavage of the pre-cleaved complement component C5 complex or the cleavage products of the complement component C5.

It is understood that inhibition of C5 cleavage prevents the assembly and activity of the cytolytic membrane attack complex (MAC) on glycosylphosphatidylinositol (GPI) adherent protein-deficient erythrocytes. In some cases, C5 inhibitors may also bind C5b, preventing C6 binding and subsequent assembly of the C5b-9 MAC.

C5 inhibitor compounds are presented in Table 1. The C5 inhibitor for use in the present invention is zilucoplan.

**Table 1. C5 inhibitors**

| **Compound** | **Company** | **Target** | **Compound type** | **Clinical study numbers** | **References** |
|---|---|---|---|---|---|
| Eculizumab (SOLIRIS^{®}) | Alexion Pharmaceuticals, Inc. | C5 | Monoclonal antibody directed against C5 protein. Inhibits C5 cleavage. | NCT01303952; NCT02093533; NCT01567085; NCT01919346; NCT01895127; NCT01399593; NCT02145182; NCT01106027; NCT02301624; NCT01997229; NCT01892345 | US Patent No. 6,355,245; 9,732,149; 9,718,880 |
| ALXN1210 | Alexion Pharmaceuticals, Inc. | C5 | Antibody | NCT02598583; NCT02605993; NCT02946463; NCT03056040; NCT02949128 | US 2016/0168237 |
| Tesidolumab/L FG316 | Novartis | C5 | Antibody | NCT02878616; NCT02763644; NCT01527500; NCT02515942; NCT02534909; NCT01526889 | US 8,241,628; US 8,883,158 |
| ALN-CC5 | Alnylam | C5 | Nucleic acid | NCT02352493 | |
| Zimura | Ophthotech | C5 | Nucleic acid | NCT02397954; NCT02686658 | |
| Coversin | Akari | C5 | Protein | NCT02591862 | |
| ALXN1007 | Alexion | C5a | Antibody | NCT02245412; NCT02128269 | |
| IFX-1 | InflaRx | C5a | Antibody | NCT02246595; NCT02866825; NCT03001622 | |
| MUBODINA^{®} | Adienne Pharma | C5 | Antibody | | US 7,999,081 |
| ALXN5500 | Alexion Pharmaceuticals, Inc. | C5 | Antibody | | |
| ISU305 | ISU ABXIS | C5 | Antibody | | |
| Long-acting coversin | Akari | C5 | Protein | | |
| SOBI005 | Swedish Orphan Biovitrum Ab | C5 | Protein | | |
| IFX-2, IFX-3 | InflaRx | C5a | Antibody | | |
| NOX-D21 | Noxxon | C5a | Spiegelmer | | |
| rEV576 | Volution Immunopharmaceu ticals | C5 | Antibody | | Penabad et al., Lupus, 2014 23(12): 1324-6 |
| ARC1005 | Novo Nordisk | C5 | Antibody | | |
| SOMAmers | SomaLogic | C5 | Antibody | | |

### Peptide-based compounds

According to the present disclosure, any amino acid-based molecule (natural or non-natural) may be termed a "polypeptide" and this term embraces "peptides," "peptidomimetics," and "proteins." "Peptides" are traditionally considered to range in size from about 4 to about 50 amino acids. Polypeptides larger than about 50 amino acids are generally termed "proteins." The polypeptide of the present invention is zilucoplan.

Cyclic polypeptides include any polypeptides that have as part of their structure one or more cyclic features such as a loop and/or an internal linkage. Cyclic polypeptides may be formed when a molecule acts as a bridging moiety to link two or more regions of the polypeptide. As used herein, the term "bridging moiety" refers to one or more components of a bridge formed between two adjacent or non-adjacent amino acids, non-natural amino acids or non-amino acids in a polypeptide. Bridging moieties may be of any size or composition. Bridging moieties may include one or more chemical bonds between two adjacent or non-adjacent amino acids, non-natural amino acids, non-amino acid residues or combinations thereof. Such chemical bonds may be between one or more functional groups on adjacent or non-adjacent amino acids, non-natural amino acids, non-amino acid residues or combinations thereof. Bridging moieties may include one or more of an amide bond (lactam), disulfide bond, thioether bond, aromatic ring, triazole ring, and hydrocarbon chain. Bridging moieties may include an amide bond between an amine functionality and a carboxylate functionality, each present in an amino acid, non-natural amino acid or non-amino acid residue side chain. The amine or carboxylate functionalities may be part of a non-amino acid residue or non-natural amino acid residue.

C5 inhibitor polypeptides may be cyclized through the carboxy terminus, the amino terminus, or through any other convenient point of attachment, such as, for example, through the sulfur of a cysteine (e.g., through the formation of disulfide bonds between two cysteine residues in a sequence) or any side-chain of an amino acid residue. Further linkages forming cyclic loops may include maleimide linkages, amide linkages, ester linkages, ether linkages, thiol ether linkages, hydrazone linkages, or acetamide linkages.

In some embodiments, zilucoplan may be synthesized on solid supports (e.g., rink amide resin) via solid phase peptide synthesis (SPPS). SPPS methods are known in the art and may be performed with orthogonal protecting groups. In some embodiments, zilucoplan may be synthesized via SPPS with Fmoc chemistry and/or Boc chemistry. Synthesized peptides may be cleaved from solid supports using standard techniques.

Peptides may be purified via chromatography [e.g., size exclusion chromatography (SEC) and/or high performance liquid chromatography (HPLC)]. HPLC may include reverse phase HPLC (RP-HPLC). Peptides may be freeze-dried after purification. Purified peptides may be obtained as pure peptide or as a peptide salt. Residual salts making up peptide salts may include, but are not limited to, trifluoroacetic acid (TFA), acetate, and/or hydrochloride. In some embodiments, zilucoplans is obtained as a peptide salt. The peptide salt may be peptide salt with TFA. Residual salts may be removed from purified peptides according to known methods (e.g., through use of desalting columns).

Cyclic C5 inhibitor polypeptides may be formed using a lactam moiety. Such cyclic polypeptides may be formed, for example, by synthesis on a solid support Wang resin using standard Fmoc chemistry. In some cases, Fmoc-ASP(allyl)-OH and Fmoc-LYS(alloc)-OH are incorporated into polypeptides to serve as precursor monomers for lactam bridge formation.

A "peptidomimetic" or "polypeptide mimetic" is a polypeptide in which the molecule contains structural elements that are not found in natural polypeptides (i.e., polypeptides comprised of only the 20 proteinogenic amino acids). Peptidomimetics may be capable of recapitulating or mimicking the biological action(s) of a natural peptide. A peptidomimetic may differ in many ways from natural polypeptides, for example through changes in backbone structure or through the presence of amino acids that do not occur in nature. In some cases, peptidomimetics may include amino acids with side chains that are not found among the known 20 proteinogenic amino acids; non-polypeptide-based bridging moieties used to effect cyclization between the ends or internal portions of the molecule; substitutions of the amide bond hydrogen moiety by methyl groups (N-methylation) or other alkyl groups; replacement of a peptide bond with a chemical group or bond that is resistant to chemical or enzymatic treatments; N- and C-terminal modifications; and/or conjugation with a non-peptidic extension (such as polyethylene glycol, lipids, carbohydrates, nucleosides, nucleotides, nucleoside bases, various small molecules, or phosphate or sulfate groups).

As used herein, the term "amino acid" includes the residues of the natural amino acids as well as non-natural amino acids. The 20 natural proteinogenic amino acids are identified and referred to herein by either the one-letter or three-letter designations as follows: aspartic acid (Asp:D), isoleucine (Ile:I), threonine (Thr:T), leucine (Leu:L), serine (Ser: S), tyrosine (Tyr:Y), glutamic acid (Glu:E), phenylalanine (Phe:F), proline (Pro:P), histidine (His:H), glycine (Gly:G), lysine (Lys:K), alanine (Ala:A), arginine (Arg:R), cysteine (Cys:C), tryptophan (Trp:W), valine (Val:V), glutamine (Gln:Q) methionine (Met:M), asparagine (Asn:N). Naturally occurring amino acids exist in their levorotary (L) stereoisomeric forms. Amino acids referred to herein are L-stereoisomers except where otherwise indicated. The term "amino acid" also includes amino acids bearing a conventional amino protecting group (e.g. acetyl or benzyloxycarbonyl), as well as natural and non-natural amino acids protected at the carboxy terminus (e.g., as a (C1-C6) alkyl, phenyl or benzyl ester or amide; or as an alpha-methylbenzyl amide). Other suitable amino and carboxy protecting groups are known to those skilled in the art (See for example, Greene, T. W.; Wutz, P. G. M., Protecting Groups In Organic Synthesis; second edition, 1991, New York, John Wiley & sons, Inc., and documents cited therein).

"Non-natural" amino acids have side chains or other features not present in the 20 naturally-occurring amino acids listed above and include, but are not limited to: N-methyl amino acids, N-alkyl amino acids, alpha, alpha substituted amino acids, beta-amino acids, alpha-hydroxy amino acids, D-amino acids, and other non-natural amino acids known in the art (See, e.g., Josephson et al., (2005) J. Am. Chem. Soc. 127: 11727-11735; Forster, A.C. et al. (2003) Proc. Natl. Acad. Sci. USA 100: 6353-6357; Subtelny et al., (2008) J. Am. Chem. Soc. 130: 6131-6136; Hartman, M.C.T. et al. (2007) PLoS ONE 2:e972; and Hartman et al., (2006) Proc. Natl. Acad. Sci. USA 103:4356-4361). Further non-natural amino acids include 1,2,3,4-tetrahydroisoquinoline-1-carboxylic acid, 1-amino-2,3-hydro-1H-indene-1-carboxylic acid, homolysine, homoarginine, homoserine, 2-aminoadipic acid, 3-aminoadipic acid, beta-alanine, aminopropionic acid, 2-aminobutyric acid, 4-aminobutyric acid, 5-aminopentanoic acid, 5-aminohexanoic acid, 6-aminocaproic acid, 2-aminoheptanoic acid, 2-aminoisobutyric acid, 3-aminoisobutyric acid, 2-aminopimelic acid, desmosine, 2,3-diaminopropionic acid, N-ethylglycine, N-ethylasparagine, homoproline, hydroxylysine, allo-hydroxylysine, 3-hydroxyproline, 4-hydroxyproline, isodesmosine, allo-isoleucine, N-methylpentylglycine, naphthylalanine, ornithine, pentylglycine, thioproline, norvaline, tert-butylglycine, phenylglycine, azatryptophan, 5-azatryptophan, 7-azatryptophan, 4-fluorophenylalanine, penicillamine, sarcosine, homocysteine, 1-aminocyclopropanecarboxylic acid, 1-aminocyclobutanecarboxylic acid, 1-aminocyclopentanecarboxylic acid, 1-aminocyclohexanecarboxylic acid, 4-aminotetrahydro-2*H*-pyran-4-carboxylic acid, (*S*)-2-amino-3-(1*H*-tetrazol-5-yl)propanoic acid, cyclopentylglycine, cyclohexylglycine, cyclopropylglycine, η-ω-methyl-arginine, 4-chlorophenylalanine, 3-chlorotyrosine, 3-fluorotyrosine, 5-fluorotryptophan, 5-chlorotryptophan, citrulline, 4-chloro-homophenylalanine, homophenylalanine, 4-aminomethyl-phenylalanine, 3-aminomethyl-phenylalanine, octylglycine, norleucine, tranexamic acid, 2-amino pentanoic acid, 2-amino hexanoic acid, 2-amino heptanoic acid, 2-amino octanoic acid, 2-amino nonanoic acid, 2-amino decanoic acid, 2-amino undecanoic acid, 2-amino dodecanoic acid, aminovaleric acid, and 2-(2-aminoethoxy)acetic acid, pipecolic acid, 2-carboxy azetidine, hexafluoroleucine, 3-Fluorovaline, 2-amino-4,4-difluoro-3-methylbutanoic acid, 3-fluoro-isoleucine, 4-fluoroisoleucine, 5-fluoroisoleucine, 4-methyl-phenylglycine, 4-ethyl-phenylglycine, 4-isopropyl-phenylglycine, (S)-2-amino-5-azidopentanoic acid (also referred to herein as "X02"), (S)-2-aminohept-6-enoic acid (also referred to herein as "X30"), (S)-2-aminopent-4-ynoic acid (also referred to herein as "X31"), (S)-2-aminopent-4-enoic acid (also referred to herein as "X12"), (*S*)-2-amino-5-(3-methylguanidino) pentanoic acid, (*S*)-2-amino-3-(4-(aminomethyl)phenyl)propanoic acid, (*S*)-2-amino-3-(3-(aminomethyl)phenyl)propanoic acid, (*S*)-2-amino-4-(2-aminobenzo[*d*]oxazol-5-yl)butanoic acid, (S)-leucinol, (S)-valinol, (S)-tert-leucinol, (*R*)-3-methylbutan-2-amine, (*S*)-2-methyl-1-phenylpropan-1-amine, and (*S*)-*N*,2-dimethyl-1-(pyridin-2-yl)propan-1-amine, (*S*)-2-amino-3-(oxazol-2-yl)propanoic acid, (*S*)-2-amino-3-(oxazol-5-yl)propanoic acid, (*S*)-2-amino-3-(1,3,4-oxadiazol-2-yl)propanoic acid, (*S*)-2-amino-3-(1,2,4-oxadiazol-3-yl)propanoic acid, (*S*)-2-amino-3-(5-fluoro-1*H-*indazol-3-yl)propanoic acid, and (*S*)-2-amino-3-(1*H*-indazol-3-yl)propanoic acid, (*S*)-2-amino-3-(oxazol-2-yl)butanoic acid, (*S*)-2-amino-3-(oxazol-5-yl) butanoic acid, (*S*)-2-amino-3-(1,3,4-oxadiazol-2-yl) butanoic acid, (*S*)-2-amino-3-(1,2,4-oxadiazol-3-yl) butanoic acid, (*S*)-2-amino-3-(5-fluoro-1*H*-indazol-3-yl) butanoic acid, and (*S*)-2-amino-3-(1*H*-indazol-3-yl) butanoic acid, 2-(2'MeOphenyl)-2-amino acetic acid, tetrahydro 3-isoquinolinecarboxylic acid and stereoisomers thereof (including, but not limited, to D and L isomers).

Additional non-natural amino acids include fluorinated amino acids wherein one or more carbon bound hydrogen atoms are replaced by fluorine. The number of fluorine atoms included can range from 1 up to and including all of the hydrogen atoms. Examples of such amino acids include but are not limited to 3-fluoroproline, 3,3-difluoroproline, 4-fluoroproline, 4,4-difluoroproline, 3,4-difluroproline, 3,3,4,4-tetrafluoroproline, 4-fluorotryptophan, 5-flurotryptophan, 6-fluorotryptophan, 7-fluorotryptophan, and stereoisomers thereof.

Further non-natural amino acids include those that are disubstituted at the α-carbon. These include amino acids in which the two substituents on the α-carbon are the same, for example α-amino isobutyric acid, and 2-amino-2-ethyl butanoic acid, as well as those where the substituents are different, for example α-methylphenylglycine and α-methylproline. Further the substituents on the α-carbon may be taken together to form a ring, for example 1-aminocyclopentanecarboxylic acid, 1- aminocyclobutanecarboxylic acid, 1-aminocyclohexanecarboxylic acid, 3-aminotetrahydrofuran-3-carboxylic acid, 3-aminotetrahydropyran-3-carboxylic acid, 4-aminotetrahydropyran-4-carboxylic acid, 3-aminopyrrolidine-3-carboxylic acid, 3-aminopiperidine-3-carboxylic acid, 4-aminopiperidinnne-4-carboxylix acid, and stereoisomers thereof.

Additional non-natural amino acids include analogs of tryptophan in which the indole ring system is replaced by another 9 or 10 membered bicyclic ring system with 0, 1, 2, 3 or 4 heteroatoms independently selected from N, O, or S. Each ring system may be saturated, partially unsaturated, or fully unsaturated. The ring system may be substituted by 0, 1, 2, 3, or 4 substituents at any substitutable atom. Each substituent may be independently selected from H, F, Cl, Br, CN, COOR, CONRR', oxo, OR, NRR'. Each R and R' may be independently selected from H, C1-C20 alkyl, or C1-C20 alkyl-O-C1-20 alkyl.

Analogs of tryptophan (also referred to herein as "tryptophan analogs") may be useful in the optimization of polypeptides or polypeptide. Tryptophan analogs may include 5-fluorotryptophan [(5-F)W], 5-methyl-O-tryptophan [(5-MeO)W], 1-methyltryptophan [(1-Me-W) or (1-Me)W], D-tryptophan (D-Trp), azatryptophan (including, but not limited to 4-azatryptophan, 7-azatryptophan and 5-azatryptophan,) 5-chlorotryptophan, 4-fluorotryptophan, 6-fluorotryptophan, 7-fluorotryptophan, and stereoisomers thereof. Except where indicated to the contrary, the term "azatryptophan" and its abbreviation, "azaTrp," as used herein, refer to 7-azatryptophan.

Modified amino acid residues include those which are chemically blocked (reversibly or irreversibly); chemically modified on their N-terminal amino group or their side chain groups; chemically modified in the amide backbone, as for example, N-methylated, D (non-natural amino acids) and L (natural amino acids) stereoisomers; or residues wherein the side chain functional groups are chemically modified to another functional group. Modified amino acids include methionine sulfoxide; methionine sulfone; aspartic acid-(beta-methyl ester), a modified amino acid of aspartic acid; N-ethylglycine, a modified amino acid of glycine; alanine carboxamide; and/or a modified amino acid of alanine. Non-natural amino acids may be purchased from Sigma-Aldrich (St. Louis, MO), Bachem (Torrance, CA) or other suppliers. Non-natural amino acids may further include any of those listed in Table 2 of US patent publication US 2011/0172126.

Abbreviations used herein include: "Ac" and "NH2" indicate acetyl and amidated termini, respectively; "Nvl" stands for norvaline; "Phg" stands for phenylglycine; "Tbg" stands for tert-butylglycine; "Chg" stands for cyclohexylglycine; "(N-Me)X" stands for the N-methylated form of the amino acid indicated by the letter or three letter amino acid code in place of variable "X" written as N-methyl-X [e.g. (N-Me)D or (N-Me)Asp stand for the N-methylated form of aspartic acid or N-methyl-aspartic acid]; "azaTrp" stands for azatryptophan; "(4-F)Phe" stands for 4-fluorophenylalanine; "Tyr(OMe)" stands for O-methyl tyrosine, "Aib" stands for amino isobutyric acid; "(homo)F" or "(homo)Phe" stands for homophenylalanine; "(2-OMe)Phg" refers to 2-O-methylphenylglycine; "(5-F)W" refers to 5-fluorotryptophan; "D-X" refers to the D-stereoisomer of the given amino acid "X" [e.g. (D-Chg) stands for D-cyclohexylglycine]; "(5-MeO)W" refers to 5-methyl-O-tryptophan; "homoC" refers to homocysteine; "(1-Me-W)" or "(1-Me)W" refers to 1-methyltryptophan; "Nle" refers to norleucine; "Tiq" refers to a tetrahydroisoquinoline residue; "Asp(T)" refers to (*S*)-2-amino-3-(1*H*-tetrazol-5-yl)propanoic acid; "(3-Cl-Phe)" refers to 3-chlorophenylalanine; "[(N-Me-4-F)Phe]" or "(N-Me-4-F)Phe" refers to N-methyl-4-fluorophenylalanine; "(m-Cl-homo)Phe" refers to meta-chloro homophenylalanine; "(des-amino)C" refers to 3-thiopropionic acid; "(alpha-methyl)D" refers to alpha-methyl L-aspartic acid; "2Nal" refers to 2-naphthylalanine; "(3-aminomethyl)Phe" refers to 3-aminomethyl-L-phenyalanine; "Cle" refers to cycloleucine; "Ac-Pyran" refers to 4-amino-tetrahydro-pyran-4-carboxylic acid; "(Lys-C16)" refers to N-ε-palmitoyl lysine; "(Lys-C12)" refers to N-ε-lauryl lysine; "(Lys-C10)" refers to N-ε-capryl lysine; "(Lys-C8)" refers to N-ε-caprylic lysine; "[*x*Xylyl(*y*, z)]" refers to the xylyl bridging moiety between two thiol containing amino acids where x may be m, p or o to indicate the use of meta-, para- or ortho-dibromoxylenes (respectively) to generate bridging moieties and the numerical identifiers, y and z, place the amino acid position within the polypeptide of the amino acids participating in the cyclization; "[cyclo(*y*,*z*)]" refers to the formation of a bond between two amino acid residues where the numerical identifiers, y and z, place the position of the residues participating in the bond; "[cyclo-olefinyl(y,z)]" refers to the formation of a bond between two amino acid residues by olefin metathesis where the numerical identifiers, y and z, place the position of the residues participating in the bond; "[cyclo-thioalkyl(y,z)]" refers to the formation of a thioether bond between two amino acid residues where the numerical identifiers, y and z, place the position of the residues participating in the bond; "[cyclotriazolyl(y,z)]" refers to the formation of a triazole ring between two amino acid residues where the numerical identifiers, y and z, place the position of the residues participating in the bond. "B20" refers to N-**ε**-(PEG2-**γ**-glutamic acid-N-α-octadecanedioic acid) lysine [also known as (1*S*,28*S*)-1-amino-7,16,25,30-tetraoxo-9,12,18,21-tetraoxa-6,15,24,29-tetraazahexatetracontane-1,28,46-tricarboxylic acid.]

### B20

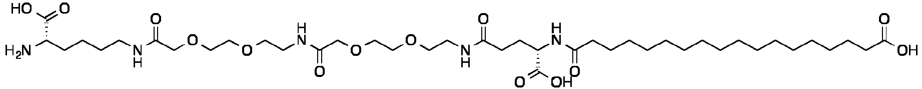

"B28" refers to N-**ε**-(PEG24-**γ**-glutamic acid-N-α-hexadecanoyl)lysine.

### B28

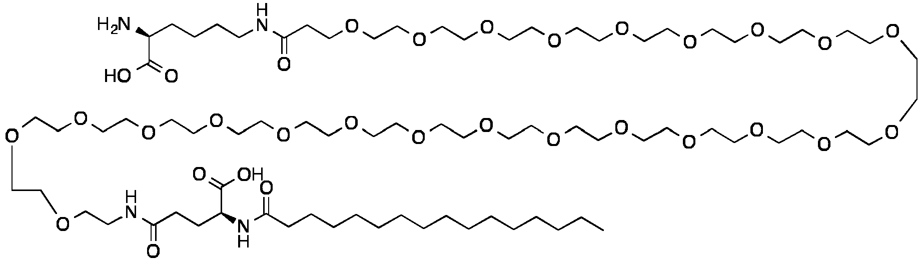

"K14" refers to N-**ε**-1-(4,4-dimethyl-2,6-dioxocyclohex-1-ylidene)-3-methylbutyl-L-lysine. All other symbols refer to the standard one-letter amino acid code.

The core amino acid sequence of zilucoplan ([cyclo(1,6)]Ac-K-V-E-R-F-D-(N-Me)D-Tbg-Y-azaTrp-E-Y-P-Chg-K; SEQ ID NO: 1) includes 15 amino acids (all L-amino acids), including 4 non-natural amino acids [N-methyl-aspartic acid or "(N-Me)D", tert-butylglycine or "Tbg", 7-azatryptophan or "azaTrp", and cyclohexylglycine or "Chg"]; a lactam bridge between K1 and D6 of the polypeptide sequence; and a C-terminal lysine reside with a modified side chain, forming a N-ε-(PEG24-γ-glutamic acid-N-α-hexadecanoyl)lysine residue (also referred to herein as "B28"). The C-terminal lysine side chain modification includes a polyethyleneglycol (PEG) spacer (PEG24), with the PEG24 being attached to an L-γ glutamic acid residue that is derivatized with a palmitoyl group.

The free acid form of zilucoplan has a molecular formula of C₁₇₂H₂₇₈N₂₄O₅₅, a molecular weight of 3562.23 Daltons (Da), and an exact mass of 3559.97 amu (see CAS Number 1841136-73-9). The tetra sodium form of zilucoplan has a molecular formula of C₁₇₂H₂₇₈N₂₄O₅₅Na₄. The chemical structure of sodium salt form of zilucoplan is shown in structure I:

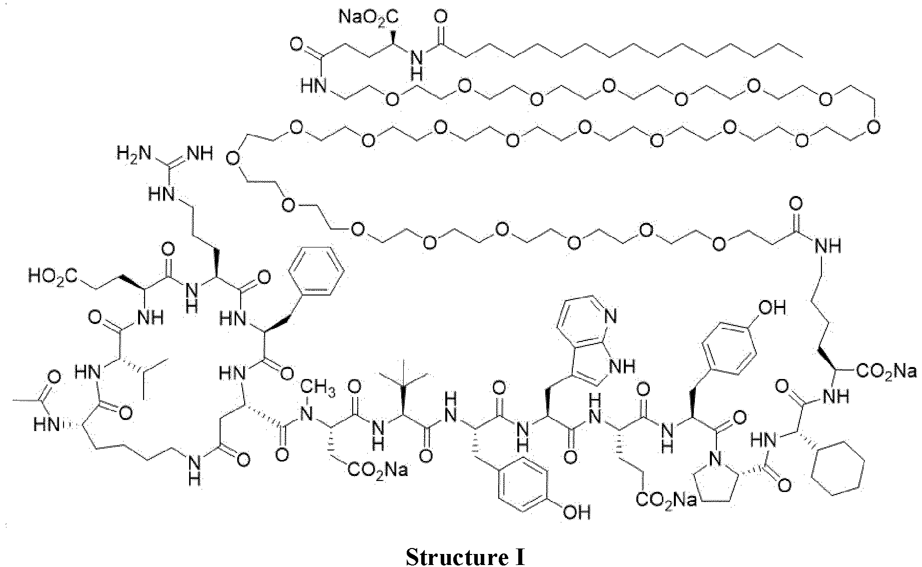

The four sodium ions in the structure are shown associated with designated carboxylates, but they may be associated with any of the acidic groups in the molecule. The zilucoplan drug substance is typically provided as the sodium salt form and is lyophilized. The free base form of zilucoplan or any pharmaceutically acceptable salt of zilucoplan are encompassed by the term *"zilucoplan".*

Metabolites of zilucoplan may include ω-hydroxylation of the palmitoyl tail. Such variants may be formed by hydroxylation of a zilucoplan precursor.

Other C5 inhibitors are listed in Table 1 of United States Publication Number US 2017/0137468. The C5 inhibitor for use in the present invention is zilucoplan.

Inhibitor binding may be assessed by determining rates of association and/or dissociation with a particular target. C5 inhibitors may demonstrate strong and rapid association with C5. Such inhibitors may further demonstrate slow rates of dissociation with C5.

In some embodiments, zilucoplan blocks the formation or generation of C5a from C5. In some case, formation or generation of C5a is blocked following activation of the alternative pathway of complement activation. In some cases, zilucoplan blocks the formation of the membrane attack complex (MAC). Such MAC formation inhibition may be due to C5 inhibitor binding to C5b subunits. C5 inhibitor binding to C5b subunits may prevent C6 binding, resulting in blockage of MAC formation. In some embodiments, this MAC formation inhibition occurs after activation of the classical, alternative, or lectin pathways.

Zilucoplan may be synthesized using chemical processes. In some cases, such synthesis eliminates risks associated with the manufacture of biological products in mammalian cell lines. In some cases, chemical synthesis may be simpler and more cost-effective than biological production processes.

In some embodiments, zilucoplan compositions may be pharmaceutical compositions that include at least one pharmaceutically acceptable excipient. In some embodiments, the pharmaceutically acceptable excipient may include at least one of a salt and a buffering agent. The salt may be sodium chloride. The buffering agent may be sodium phosphate. Sodium chloride may be present at a concentration of from about 0.1 mM to about 1000 mM. In some cases, sodium chloride may be present at a concentration of from about 25 mM to about 100 mM. Sodium phosphate may be present at a concentration of from about 0.1 mM to about 1000 mM. In some cases, sodium phosphate is present at a concentration of from about 10 mM to about 100 mM.

In some embodiments, zilucoplan compositions may include from about 0.01 mg/mL to about 4000 mg/mL of zilucoplan. In some cases, zilucoplan is present at a concentration of from about 1 mg/mL to about 400 mg/mL.

Zilucoplan binds to C5 and inhibits cleavage of C5 by canonical complement pathway convertases as described in International Publication Number WO2018106859. Zilucoplan additionally binds C5b, preventing the formation of the membrane attack complex induced by non-canonical cleavage of C5. Zilucoplan binding and inhibitory activities are not affected by the presence of clinically-relevant human C5 polymorphisms (including p.R885>H/C). Unlike eculizumab, an anti-C5 monoclonal antibody inhibitor, zilucoplan does not bind to surface-bound C5b-9 or soluble membrane attack complex (sC5b-9).

### Isotopic variations

Compounds of the present disclosure may include one or more atoms that are isotopes. As used herein, the term "isotope" refers to a chemical element that has one or more additional neutrons. In some embodiments, compounds of the present disclosure may be deuterated. As used herein, the term "deuterated" refers to a substance that has had one or more hydrogen atoms replaced by deuterium isotopes. Deuterium isotopes are isotopes of hydrogen. The nucleus of hydrogen contains one proton while deuterium nuclei contain both a proton and a neutron. Compounds and compositions of the present disclosure may be deuterated in order to change a physical property, such as stability, or to allow for use in diagnostic and experimental applications.

### II. Methods and uses

### Therapeutic indications

As used herein, the term "therapeutic indication" refers to any symptom, condition, disorder, or disease that may be alleviated, stabilized, improved, cured, or otherwise addressed by some form of treatment or other therapeutic intervention (e.g., through complement inhibitor administration). Examples of therapeutic indications include inflammatory indications, wounds, injuries, autoimmune indications, vascular indications, neurological indications, kidney-related indications, ocular indications, cardiovascular indications, pulmonary indications, and pregnancy-related indications. Therapeutic indications associated with complement activity and/or dysfunction are referred to herein as "complement-related indications." The therapeutic indication of the present invention is ALS.

Complement-related indications are presented in United States Patent Number 10,106,579.

As used herein the terms "treat," "treatment," and the like, refer to relief from or alleviation of pathological processes. In the context of the present disclosure insofar as it relates to any of the other conditions recited herein below, the terms "treat," "treatment," and the like mean to relieve or alleviate at least one symptom associated with such condition, or to slow or reverse the progression or anticipated progression of such condition.

By "lower" or "reduce" in the context of a disease marker or symptom is meant a significant decrease in such a level, often statistically significant. The decrease may be, for example, at least 10%, at least 20%, at least 30%, at least 40% or more, and is preferably down to a level accepted as within the range of normal for an individual without such a disorder.

By "increase" or "raise" in the context of a disease marker or symptom is meant a significant rise in such level, often statistically significant. The increase may be, for example, at least 10%, at least 20%, at least 30%, at least 40% or more, and is preferably up to a level accepted as within the range of normal for an individual without such disorder.

Efficacy of treatment or amelioration of disease can be assessed, for example by measuring disease progression, disease remission, symptom severity, reduction in pain, quality of life, dose of a medication required to sustain a treatment effect, level of a disease marker or any other measurable parameter appropriate for a given disease being treated or targeted for prevention. It is well within the ability of one skilled in the art to monitor efficacy of treatment or prevention by measuring any one of such parameters, or any combination of parameters. In connection with the administration of a polypeptide or pharmaceutical composition thereof, "effective against" a disease or disorder indicates that administration in a clinically appropriate manner results in a beneficial effect for at least a fraction of patients, such as an improvement of symptoms, a cure, a reduction in disease load, reduction in tumor mass or cell numbers, extension of life, improvement in quality of life, a reduction in the need for blood transfusions or other effect generally recognized as positive by medical doctors familiar with treating the particular type of disease or disorder.

A treatment or preventive effect is evident when there is a significant improvement, often statistically significant, in one or more parameters of disease status, or by a failure to worsen or to develop symptoms where they would otherwise be anticipated. As an example, a favorable change of at least 10% in a measurable parameter of disease, and preferably at least 20%, 30%, 40%, 50% or more may be indicative of effective treatment. Efficacy for a given compound or composition may also be judged using an experimental animal model for the given disease as known in the art. When using an experimental animal model, efficacy of treatment is evidenced when a statistically significant modulation in a marker or symptom is observed.

Zilucoplan and additional therapeutic agents can be administered in combination. Such combinations may be in the same composition, or the additional therapeutic agents can be administered as part of a separate composition or by another method described herein.

As used herein, the term "tissue-penetrating" refers to a property characterized by tissue permeability. Agents with enhanced tissue-penetration may demonstrate better distribution in tissues when compared to agents with less or no tissue-penetration. Tissue penetration may be assessed by ability to cross basement membranes. As used herein, the term "basement membrane" refers to an extracellular matrix (ECM) protein layer separating endothelial cells from underlying tissues. Tissue penetration assessments may be done *in vivo* or *in vitro* and may include the use of basement membrane models. Such models may include measuring compound diffusion across artificial basement membranes. Such models may include the use of upper and lower reservoirs separated by an artificial basement membrane. Artificial basement membranes may include any of the ECM gel membranes described in Arends, F. et al. 2016. IntechOpen, DOI: 10.5772/62519. ECM gel membranes may be prepared to include matrix components mimicking those found in the basal lamina of neuromuscular junctions. In some models, compounds being tested are introduced to upper reservoirs and compound diffusion is detected in lower reservoirs.

Tissue penetration assessment may include visual assessments e.g., through use of fluorescent labels to visualize analyte movement across basement membranes. Some assessments may include biochemical analysis of samples obtained from the penetrated side of a basement membrane.

Compound permeability may be determined using quantitative whole body analysis (QWBA). QWBA is a form of analysis that uses radiography to assess distribution of radiolabeled analytes. Radiolabeled compounds can be administered to subjects and tissue distribution of the compounds is analyzed over time.

Zilucoplan is a tissue-penetrating C5 inhibitor (see International Publication Number WO2020086506). Contacting tissues with the tissue-penetrating C5 inhibitors may include administering tissue-penetrating C5 inhibitors to tissues as part of a formulation. Such formulations may be administered by subcutaneous injection. Tissue-penetrating C5 inhibitors may be able to penetrate basement membranes. Basement membrane permeability of polypeptide tissue-penetrating C5 inhibitors may be greater than basement membrane permeability of larger proteins, such as antibodies. Such advantages may be due to restrictively large size of proteins and antibodies. Zilucoplan basement membrane permeability may be from about 3-fold to about 5-fold greater than basement membrane permeability of eculizumab, offering advantages over eculizumab for inhibiting C5 activity in tissues and treating related complement-related indications. In some embodiments, zilucoplan permeability enhances distribution in one or more of lung, heart, muscle, small intestine, large intestine, spleen, liver, bone, stomach, lymph node, fat, brain, pancreas, testes, and thymus, in comparison to eculizumab.

### Neuromuscular inflammatory indications

As used herein, the term "neuromuscular inflammatory indication" refers to inflammatory indications that involve components of the nervous system, muscular system, and areas where these two systems overlap or integrate. Neuromuscular inflammation may be upregulated during the proteolytic cascade of the complement system. Although inflammation may have beneficial effects, excess inflammation may lead to a variety of pathologies (Markiewski et al. 2007. Am J Pathol. 17: 715-27).

### Amyotrophic lateral sclerosis (ALS)

ALS is a neuroinflammatory indication. ALS, also referred to as Lou Gehrig's disease, is a progressive neurodegenerative disease with upper and lower motor neuron loss in the primary motor cortex, brainstem, and spinal cord. Cortical and spinal cord motor neurons normally transmit signals from brain to muscles during voluntary movement. Neuronal loss disrupts these signals resulting in gradual weakening and/or muscle atrophy and disruption of voluntary movement capability. In addition to persistent weakness, patients also experience painful muscle cramping and limb spasticity. Affected individuals eventually lose muscle strength, mobility, and critical neuromotor functions (ability to speak, eat, swallow, and breathe). With time, patients typically require breathing assistance and most die from respiratory failure within 2-5 years of diagnosis. Only 5-10% of cases are thought to be familial, while 90-95% of cases are sporadic.

Approximately 20 gene mutations have been identified that lead to familial cases. These include, but are not limited to, Cu²⁺/Zn²⁺ superoxide dismutase (SOD1), TAR DNA binding protein-43 (TDP-43), Fused in Sarcoma/Translocated in Sarcoma (FUS), Angiogenin (ANG), Ataxin-2 (ATXN2), valosin containing protein (VCP), Optineurin (OPTN), and noncoding GGGGCC hexanucleotide repeat expansion in chromosome 9 open reading frame 72 (C9ORF72). In some cases, patients with ALS also develop frontotemporal dementia (FTD-ALS).

ALS diagnosis may be carried out according to revised El Escorial criteria as described in Brooks RB, et al. Amyotroph Lateral Scler Other Motor Neuron Disord. 2000 Dec; 1(5):293-9 and Ludolph, A. et al., ALS and FD, 2015. Early Online: 1-2. According to such analysis, subject upper motor neuron (UMN) and lower motor neuron (LMN) impairment is assessed in bulbar, cervical, thoracic, and lumbosacral regions of the body. UMN assessment includes analysis of spasticity and hyperreflexivity. LMN assessment includes assessment of weakness, atrophy, and fasciculation. Subjects with signs of UMN and LMN impairment in at least three regions tested are diagnosed as having "definite" ALS. Subjects with signs of UMN and LMN impairment in at least two regions tested are diagnosed as having "probable" ALS. Subjects with UMN and LMN signs in one region with evidence by electromyography (EMG) of LMN involvement in another region are characterized as having "laboratory results-supported probable" ALS. Subjects with signs of UMN and LMN impairment in one region tested or have UMN impairment alone in two or more regions are characterized as having "possible" ALS. Subjects with signs of LMN impairment alone in two or more regions tested are characterized as having "suspected" ALS.

Current treatments are not curative and are used to temporarily alleviate or slow the progression of symptoms. Such treatments include Riluzole, which is used to reduce pathological glutamate response. Life-span expansion reported with this treatment has not exceeded three months and only with early stage treatment (Bensimon G et al., J Neurol. 2002, 249, 609-615). Other treatments include Radicava (edaravone), which has been shown to slow disease progression, but not cure the disease.

Complement cascade activation is evident in peripheral and central ALS pathophysiology and is closely associated with ongoing neuroinflammatory processes. Complement activation may temporally precede motor end plate degeneration in the periphery. Complement inhibitor use may be used to block or slow this process. Recent studies have identified MAC deposition in spinal and cerebral neuronal cells, glial cells, and neuromuscular junctions of ALS patients. These cells produce complement proteins C1q and C4 involved in classical pathway activation. C5a receptor (C5aR1) upregulation has also been shown in motorneurons from ALS postmortem donors and SOD1G93A transgenic animals used in ALS disease models. Further, C5aR1 knockout and inhibition have been shown to prolong animal survival in ALS disease models.

ALS treatment with zilucoplan may be carried out to reduce, prevent, stabilize, and/or reverse one or more effect of ALS experienced by a subject having or suspected of having ALS. In some embodiments, treatments of the present disclosure include administering zilucoplan in combination with other therapeutic agents and/or therapeutic regimen.

Zilucoplan may be administered at a dose of from about 0.1 mg/kg to about 0.3 mg/kg. Zilucoplan may be administered daily. Zilucoplan may be administered by subcutaneous injection. The subcutaneous injection may include the use of an autoinjection device. Autoinj ection devices may include BD UL TRASAFE PLUS^{™} autoadministration devices (BD, Franklin Lakes, NJ).

Treatment of ALS may include assessments of treatment efficacy and/or other results of treatment. Such assessments may be carried out before, during, and/or after treatment. Assessments may be carried out to monitor changes in patient symptoms and/or neuromotor functions. Assessments may be carried out during clinical visits or at home. Some assessments may be carried out by subjects undergoing treatment, referred to herein as "self-assessments." Self-assessments may be carried out with the use of a smart device (e.g., smart watch, mobile phone, computer, etc.). Measures of treatment efficacy may include, but are not limited to, assessment of ALS Functional Rating Scale - Revised (ALSFRS-R); assessment of vital capacity; assessment by hand-held dynamometry (HHD); assessment by bilateral hand grip; and voice analysis.

Assessments carried out with ALS treatment methods may include physical and/or neurological examinations. Some assessments may include vital sign assessments. Some assessments may include height and weight assessments. Some assessments may include clinical laboratory assessments. Some assessments may include Edinburgh Cognitive and Behavioral ALS Screen (ECAS). Some assessments may include Columbia Suicide Severity Rating Scale (C-SSRS).

Assessments carried out with ALS treatment methods may include collection of one or more samples from subjects receiving treatment. Such subject samples may include biological samples. Samples may include, but are not limited to, blood samples, plasma samples, serum samples, urine samples, spinal fluid samples, or cerebrospinal fluid (CSF) samples. Some subject samples may include DNA. In some embodiments, subject samples are analyzed for one or more biomarkers. Biomarkers may include biomarkers of neurofilament processing and/or axonal neurodegeneration (referred to herein as "neuroaxonal degeneration biomarkers"). Such biomarkers may include, but are not limited to, phosphorylated neurofilament heavy chain (pNfH) and neurofilament light chain (NfL). Biomarkers may include neurotrophin receptor p75 extracellular domain (p75ECD). In some embodiments, biomarkers include biomarkers of neuroinflammation (referred to herein as "neuroinflammation biomarkers"). Neuroinflammation biomarkers may include, but are not limited to, monocyte chemoattractant protein-1 (MCP-1), chitotriosidase-1 (CHIT 1) and chitinase-3-like protein 1 (YKL-40). Biomarkers may include complement component C5 or biomarkers of complement activity (referred to herein as "complement activity biomarkers"). Complement activity biomarkers may include cleavage products of C5. Complement activity biomarkers may include, but are not limited to, C5a, C5b-9, soluble C5b-9 (sC5b-9), C5b6 complex, C3a, C4a, Ba, Bb, Factor I, and Factor H.

Biomarkers may be detected in subjects or biological samples obtained from subjects according to standard methods. Biomarkers may be detected by immunoassay. As used herein, the term "immunoassay" refers to any form of scientific analysis utilizing antibodies (e.g., for binding and/or detecting antibody-specific targets). Immunoassays may include enzyme-linked immunoassays (ELISAs). Immunoassays may be conducted with solid (e.g., using a fixed surface, plate or well) or solution-based (e.g., using beads or other antibody-coated particles in solution) platforms. Biomarkers may be detected using surface plasmon resonance (SPR) based platforms. Complement activity biomarker analysis may be carried out using commercial assays or kits (e.g., single or multi-plex analysis assays from Quidel Corporation, San Diego, CA).

Subject biomarkers assessments may be used to prepare an ALS biomarker profile. As used herein, the term "ALS biomarker profile" refers to a set of biomarker assessments, wherein the biomarkers are related to ALS (e.g., due to correlation with disease or disease outcome). ALS biomarker profiles may be used to assess disease severity. Changes in ALS biomarker profiles over time may be used to monitor changes in disease state (i.e., stabilization, improvement, or decline). Stable ALS biomarker profile (i.e., no change over time) may be used to indicate no change in disease state. Change in ALS biomarker profile may indicate improving or declining disease state. Change in ALS biomarker profile over time may be used to determine rate of improving or declining disease state.

In some embodiments, subjects exhibit elevated ALS disease severity and/or ALS disease severity that is increasing at a specific rate prior to complement inhibitor treatment. Treatment of such subjects with zilucoplan may stabilize or reduce ALS disease severity and/or reduce a specific rate at which subject ALS disease severity is increasing. Change in subject ALS disease severity may be measured by ALSFRS-R.

In some embodiments, subjects exhibit decreased respiratory function and/or respiratory function that is declining at a specific rate prior to complement inhibitor treatment. Treatment of such subjects with zilucoplan may stabilize or improve subject respiratory function and/or reduce the specific rate at which subject respiratory function is declining. Change in subject respiratory function may be assessed by slow vital capacity (SVC).

In some embodiments, subjects exhibit decreased muscle strength and/or muscle strength that is declining at a specific rate prior to zilucoplan treatment. Treatment of such subjects with zilucoplan may stabilize or improve subject muscle strength and/or reduce the specific rate at which subject muscle strength is declining. Change in subject muscle strength may be assessed by hand held dynamometry (HHD).

In some embodiments, subjects exhibit decreased speech capability and/or speech capability that is declining at a specific rate prior to treatment. Treatment of such subjects with zilucoplan may stabilize or improve subject speech capability and/or reduce the specific rate at which subject speech capability is declining. Subject speech capability may be assessed by assessment of one or more of articulatory precision, speaking rate, percent of speech signal voiced, phonation length, consonant length, and nasality.

In some embodiments, ALS is treated in subjects having or suspected of having myasthenia gravis or generalized myasthenia gravis (referred to herein collectively as "MG") Such treatments may be carried out to reduce, prevent, stabilize, and/or reverse one or more effect of ALS and/or MG experienced by a subject. Subjects having or suspected of having MG may be identified by positive testing for at least one autoantibody. The at least one autoantibody may include one or more of anti-acetylcholine receptor antibody, anti-LDL receptor related protein 4 (LRP4), anti-agrin, and anti-muscle associated receptor tyrosine kinase. Methods of detecting such autoantibodies in subjects may be carried out according to methods known in the art (e.g., see Rivner et al., 2017. Muscle Nerve. 55(3):430-2; Takahashi et al., 2016. BMC Neurology. 16:229; Tzartos et al., 2014. Ann Clin Transl Neurol. 1(2):80-7; Amador et al., 2016. Neuromuscul Disord. 26(6):342-6; and Mantovani, et al., 2014. J Neuroimmunol. 2014. 276(1-2):213-8).

Subject ALS disease severity may be elevated and/or may be increasing at a specific rate prior to treatment. Treatment with compositions according to the present disclosure may stabilize or reduce subject ALS disease severity and/or reduce the specific rate at which subject ALS disease severity is increasing. Changes in subject ALS disease severity may be measured by ALSFRS-R. Subject respiratory function may be decreased and/or may be declining at a specific rate prior to treatment. Treatment with the compositions may stabilize or improve subject respiratory function and/or reduce the specific rate at which subject respiratory function is declining. Changes in subject respiratory function may be assessed by SVC. Subject muscle strength may be decreased and/or declining at a specific rate prior to treatment. Compositions used in treatments may stabilize or improve subject muscle strength and/or reduce the specific rate at which subject muscle strength is declining. Changes in subject muscle strength may be measured by HHD. Compositions may be used to stabilize or improve subject speech capability and/or rate of subject speech capability decline. Subject speech capability may include one or more of articulatory precision, speaking rate, percent of speech signal voiced, phonation length, consonant length, and nasality. Compositions may be used to stabilize or improve subject ALS biomarker profiles and/or rate of subject ALS biomarker profile change. Subject ALS biomarker profiles may include one or more of pNfH, NfL, C5, and a complement activity biomarker. Complement activity biomarkers may include, but are not limited to, C5a, C5b-9, soluble C5b-9, C3a, C4a, Ba, Bb, Factor I, and Factor H. Complement activity biomarkers may be detected in subject CSF, plasma, and/or serum. Dose and/or regimen of compositions may be adjusted based on complement activity biomarker levels detected in CSF, plasma, and/or serum samples obtained from subjects after prior composition administrations. Complement activity biomarkers detected in CSF, plasma, and/or serum samples obtained from subjects after prior composition administration may include, but are not limited to, C5a, C5b-9, soluble C5b-9, C3a, C4a, Ba, Bb, Factor I, and Factor H. Subjects may have or may be suspected of having myasthenia gravis. Such subjects may be positive for at least one autoantibody (e.g., anti-acetylcholine receptor antibody, anti-LDL receptor related protein 4, anti-agrin, and/or anti-muscle associated receptor tyrosine kinase). Subjects may have impaired blood brain barrier function (e.g., as assessed by subject albumin quotient). Subjects may be determined to have definite ALS or probable ALS based on revised El Escorial criteria.

In some embodiments, subjects to be treated have or are suspected of having impaired blood brain barrier function (e.g., as a consequence of elevated complement activity). Assessment of blood brain barrier (BBB) impairment may be carried out by measuring albumin levels in subject CSF and plasma serum. The ratio of CSF albumin level to plasma serum albumin level is referred to as the "albumin quotient" or "albumin-Q." Increased BBB impairment leads to increased entry of albumin from the plasma compartment into the CSF, which increases the albumin quotient. Albumin quotient values greater than 9 are indicative of BBB impairment. In some embodiments, subjects may have BBB impairment (e.g., as assessed by albumin quotient determination).

Subjects with ALS according to the present disclosure may include subjects with definite or probable ALS, as determined by revised El Escorial criteria.

Diagnosis of ALS may be based on complement activity detected and/or quantified in association with subject biological samples. Complement activity detection and/or quantification may include detecting and/or quantifying complement activity biomarkers. Such complement activity biomarkers may include, but are not limited to, C5a, C5b-9, soluble C5b-9, C3a, C4a, Ba, Bb, Factor I, and Factor H. Complement activity biomarkers may be detected and/or quantified in subject CSF, plasma, and/or serum.

ALS may be monitored by detecting and/or quantifying complement activity associated with such subjects (or biological samples derived therefrom) at initial or earlier time points or time periods and comparing to results of similar analyses from subsequent time points or periods. Such comparisons may be used to monitor ALS in subjects by assessing changes in complement activity between initial/earlier time points or periods and the subsequent time points or periods. Complement activity detection and/or quantification may include detecting and/or quantifying complement activity biomarkers. The complement activity biomarkers may include, but are not limited to C5a, C5b-9, soluble C5b-9, C3a, C4a, Ba, Bb, Factor I, and Factor H. Complement activity biomarkers may be detected and/or quantified in subject CSF, plasma, and/or serum. Subjects with definite or probable ALS, as determined by revised El Escorial criteria, may be monitored.

Subjects with ALS or suspected of having ALS may be stratified into two or more groups. Such methods may include detecting and/or quantifying complement activity associated with subjects (or biological sample therefrom) and assigning each subject to at least one of the two or more groups based on complement activity detected and/or quantified. Complement activity detection and/or quantification may include detecting and/or quantifying complement activity biomarkers. Such complement activity biomarker may include, but are not limited to, C5a, C5b-9, soluble C5b-9, C3a, C4a, Ba, Bb, Factor I, and Factor H. Complement activity biomarkers may be detected and/or quantified in subject CSF, plasma, and/or serum. Subjects may be assigned to treatment groups based on CSF, plasma, and/or serum complement activity biomarker levels. As used herein, the term "treatment group" refers to a group of patients or subjects characterized by selection, eligibility, suitability, or otherwise designated for a particular treatment (e.g., medical treatment). Subjects may be assigned to treatment groups based on complement activity biomarker levels that are elevated in comparison to CSF, plasma, and/or serum complement activity biomarker levels in subjects without ALS. Treatment groups may be characterized by eligibility for zilucoplan treatment.

Selecting subjects for treatment may include assessing the presence or level of biomarkers in subject samples. Such biomarkers may include, but are not limited to, neuroaxonal degeneration biomarkers, neuroinflammation biomarkers, and complement activity biomarkers. Subjects may be selected for complement inhibitor treatment based on the presence or level of biomarkers assessed in subject samples. Subject samples may include plasma and/or CSF samples. Neuroaxonal degeneration biomarkers may include, but are not limited to, pNfH and/or NfL. Neuroinflammation biomarkers may include, but are not limited to, MCP-1, CHIT1, and YKL-40. Complement activity biomarkers may include, but are not limited to, C5a, C5b-9, sCSb-9, CSb6 complex, C3a, C4a, Ba, Bb, Factor I, and Factor H. Levels of one or more of Nfl, pNfH, CHIT1, C5a, and sCSb-9 may be elevated in subject samples relative to levels in subject samples from subjects without ALS (non-ALS subjects). Such subject samples may include CSF. Levels of C3a, Bb, and/or Ba may be elevated in CSF samples from subjects with ALS or suspected of having ALS, where subject BBB function is impaired [e.g., as determined by albumin quotient analysis (CSF albumin:plasma serum albumin ratio)]. Such levels may be elevated relative to levels in non-ALS subject CSF samples. Levels of C3a may be elevated in plasma samples from ALS subject with impaired BBB relative to levels in non-ALS subject plasma.

A subject may be selected according to any of the methods described above (e.g., by assessing the presence or level of neuroaxonal degeneration biomarkers, neuroinflammation biomarkers, and/or complement activity biomarkers in a subject sample).

In some embodiments, the presence or level of biomarkers in subject samples is assessed and the subjects for treatment are selected based on the assessment. Biomarkers may include one or more of neuroaxonal degeneration biomarkers, neuroinflammation biomarkers, and complement activity biomarkers. Subject samples may include plasma and/or CSF samples. Neuroaxonal degeneration biomarkers may include pNfH and/or NfL. Neuroinflammation biomarkers may include MCP-1, CHIT1, and/or YKL-40. Complement activity biomarkers may include C5a, C5b-9, sCSb-9, CSb6 complex, C3a, C4a, Ba, Bb, Factor I, and/or Factor H. Biomarker levels may be elevated in ALS subject samples (e.g., CSF samples) relative to levels in samples from subjects without ALS. Subject samples may be obtained from subjects with impaired blood brain barrier function (e.g., as assessed by albumin quotient analysis). C3a, Bb, and/or Ba may be elevated in subject samples (e.g., plasma samples) relative to levels in samples from subjects without ALS. C3a levels may be elevated in subject samples relative to levels in samples from subjects without ALS.

Dose and/or regimen of zilucoplan administered to subjects according to treatments described herein may be determined based on levels of complement activity biomarkers detected in CSF, plasma, and/or serum obtained from subjects prior to treatment. Such complement activity biomarkers may be detected in CSF, plasma, and/or serum obtained from subjects prior to treatment may include, but are not limited to, C5a, C5b-9, soluble C5b-9, C3a, C4a, Ba, Bb, Factor I, and Factor H. Dose and/or regimen of zilucoplan administration may be adjusted after one or more prior administrations based on complement activity biomarker levels detected in CSF, plasma, and/or serum obtained from subjects after one or more prior administration.

Levels of C5a, sC5b-9, and/or Bb in subject CSF may be assessed and used for one or more of ALS diagnosis, subject stratification (e.g., into a specific treatment group), ALS monitoring, and determining dose and/or regimen of zilucoplan treatment. Such assessment may include comparison to levels from healthy subjects (without ALS). Levels of complement activity biomarkers (e.g., C5a, C5b-9, soluble C5b-9, C3a, C4a, Ba, Bb, Factor I, and Factor H) in subject serum may be assessed and used for one or more of ALS diagnosis, subject stratification (e.g., into a specific treatment group), ALS monitoring, and determining dose and/or regimen of zilucoplan treatment. Such assessment may include comparison to levels from healthy subjects (without ALS).

Zilucoplan inhibits C5a formation in a dose-dependent manner upon activation of the classical pathway and inhibits C5b formation (as measured by C5b-9 or MAC deposition on a complement activating surface) upon activation of the classical and alternative complement pathways. (United States Patent Number 9,937,222). Zilucoplan administration may be subcutaneous (SC) administration. Zilucoplan may be administered at a dose of from about 0.01 mg/kg (mg zilucoplan/kg subject body weight) to about 1.0 mg/kg, from about 0.02 mg/kg to about 2.0 mg/kg, from about 0.05 mg/kg to about 3.0 mg/kg, from about 0.10 mg/kg to about 4.0 mg/kg, from about 0.15 mg/kg to about 4.5 mg/kg, from about 0.20 mg/kg to about 5.0 mg/kg, from about 0.30 mg/kg to about 7.5 mg/kg, from about 0.40 mg/kg to about 10 mg/kg, from about 0.50 mg/kg to about 12.5 mg/kg, from about 0.1 mg/kg to about 0.6 mg/kg, from about 1.0 mg/kg to about 15 mg/kg, from about 2.0 mg/kg to about 20 mg/kg, from about 5.0 mg/kg to about 25 mg/kg, from about 10 mg/kg to about 45 mg/kg, from about 20 mg/kg to about 55 mg/kg, from about 30 mg/kg to about 65 mg/kg, from about 40 mg/kg to about 75 mg/kg, from about 50 mg/kg to about 150 mg/kg, from about 100 mg/kg to about 250 mg/kg, from about 200 mg/kg to about 350 mg/kg, from about 300 mg/kg to about 450 mg/kg, from about 400 mg/kg to about 550 mg/kg, or from about 500 mg/kg to about 1000 mg/kg.

In some embodiments, zilucoplan may be administered at a dose of from about 0.10 mg/kg to about 0.42 mg/kg.

Treatments of the present disclosure may include administering zilucoplan at a daily dose of from about 0.1 mg/kg to about 0.3 mg/kg. In some embodiments, zilucoplan is administered at a daily dose of 0.3 mg/kg.

Zilucoplan administration may be by self-administration. Zilucoplan administration may include the use of prefilled syringes. Self-administration may include the use of self-administration devices. Self-administration devices may include or be incorporated with prefilled syringes.

Zilucoplan may be provided in solution. Zilucoplan solutions may include aqueous solutions. Zilucoplan solutions may include phosphate-buffered saline (PBS). Zilucoplan solutions may be preservative-free. Zilucoplan may be present in solutions at a concentration of from about 0.01 mg/mL to about 1 mg/mL, from about 0.05 mg/mL to about 2 mg/mL, from about 1 mg/mL to about 5 mg/mL, from about 2 mg/mL to about 10 mg/mL, from about 4 mg/mL to about 16 mg/mL, from about 5 mg/mL to about 20 mg/mL, from about 8 mg/mL to about 24 mg/mL, from about 10 mg/mL to about 30 mg/mL, from about 12 mg/mL to about 32 mg/mL, from about 14 mg/mL to about 34 mg/mL, from about 16 mg/mL to about 36 mg/mL, from about 18 mg/mL to about 38 mg/mL, from about 20 mg/mL to about 40 mg/mL, from about 22 mg/mL to about 42 mg/mL, from about 24 mg/mL to about 44 mg/mL, from about 26 mg/mL to about 46 mg/mL, from about 28 mg/mL to about 48 mg/mL, from about 30 mg/mL to about 50 mg/mL, from about 35 mg/mL to about 55 mg/mL, from about 40 mg/mL to about 60 mg/mL, from about 45 mg/mL to about 75 mg/mL, from about 50 mg/mL to about 100 mg/mL, from about 60 mg/mL to about 200 mg/mL, from about 70 mg/mL to about 300 mg/mL, from about 80 mg/mL to about 400 mg/mL, from about 90 mg/mL to about 500 mg/mL, or from about 100 mg/mL to about 1000 mg/mL.

In some embodiments, self-administration devices include zilucoplan solutions. Self-administration devices may include zilucoplan solution volumes of from about 0.010 mL to about 0.500 mL, from about 0.050 mL to about 0.600 mL, from about 0.100 mL to about 0.700 mL, from about 0.150 mL to about 0.810 mL, from about 0.200 mL to about 0.900 mL, from about 0.250 mL to about 1.00 mL, from about 0.300 mL to about 3.00 mL, from about 0.350 mL to about 3.50 mL, from about 0.400 mL to about 4.00 mL, from about 0.450 mL to about 4.50 mL, from about 0.500 mL to about 5.00 mL, from about 0.550 mL to about 10.0 mL, from about 0.600 mL to about 25.0 mL, from about 0.650 mL to about 50.0 mL, from about 0.700 mL to about 60.0 mL, from about 0.750 mL to about 75.0 mL, from about 0.800 mL to about 80.0 mL, from about 0.850 mL to about 85.0 mL, from about 0.900 mL to about 90.0 mL, from about 0.950 mL to about 95.0 mL, from about 1.00 mL to about 100 mL, from about 2.00 mL to about 200 mL, from about 5.00 mL to about 500 mL, from about 10.0 mL to about 750 mL, from about 25.0 mL to about 800 mL, from about 50.0 mL to about 900 mL, or from about 100 mL to about 1000 mL.

### Formulations

In some embodiments, compounds or compositions, e.g., pharmaceutical compositions, of the present disclosure are formulated in aqueous solutions. In some cases, aqueous solutions further include one or more salt and/or one or more buffering agent. Salts may include sodium chloride which may be included at concentrations of from about 0.05 mM to about 50 mM, from about 1 mM to about 100 mM, from about 20 mM to about 200 mM, or from about 50 mM to about 500 mM. Further solutions may include at least 500 mM sodium chloride. In some cases, aqueous solutions include sodium phosphate. Sodium phosphate may be included in aqueous solutions at a concentration of from about 0.005 mM to about 5 mM, from about 0.01 mM to about 10 mM, from about 0.1 mM to about 50 mM, from about 1 mM to about 100 mM, from about 5 mM to about 150 mM, or from about 10 mM to about 250 mM. In some cases, at least 250 mM sodium phosphate concentrations are used.

Compositions of the present disclosure may include zilucoplan at a concentration of from about 0.001 mg/mL to about 0.2 mg/mL, from about 0.01 mg/mL to about 2 mg/mL, from about 0.1 mg/mL to about 10 mg/mL, from about 0.5 mg/mL to about 5 mg/mL, from about 1 mg/mL to about 20 mg/mL, from about 15 mg/mL to about 40 mg/mL, from about 25 mg/mL to about 75 mg/mL, from about 50 mg/mL to about 200 mg/mL, or from about 100 mg/mL to about 400 mg/mL. In some cases, compositions include zilucoplan at a concentration of at least 400 mg/mL.

Compositions of the present disclosure may include zilucoplan at a concentration of approximately, about or exactly any of the following values: 0.001 mg/mL, 0.2 mg/mL, 0.01 mg/mL, 2 mg/mL, 0.1 mg/mL, 10 mg/mL, 0.5 mg/mL, 5 mg/mL, 1 mg/mL, 20 mg/mL, 15 mg/mL, 40 mg/mL, 25 mg/mL, 75 mg/mL, 50 mg/mL, 200 mg/mL, 100 mg/mL, or 400 mg/mL. In some cases, compositions include zilucoplan at a concentration of at least 40 mg/mL.

In some embodiments, compositions of the present disclosure include aqueous compositions including at least water and a zilucoplan. Aqueous compositions may further include one or more salt and/or one or more buffering agent. In some cases, aqueous compositions include water, zilucoplan, a salt, and a buffering agent.

Aqueous zilucoplan formulations may have pH levels of from about 2.0 to about 3.0, from about 2.5 to about 3.5, from about 3.0 to about 4.0, from about 3.5 to about 4.5, from about 4.0 to about 5.0, from about 4.5 to about 5.5, from about 5.0 to about 6.0, from about 5.5 to about 6.5, from about 6.0 to about 7.0, from about 6.5 to about 7.5, from about 7.0 to about 8.0, from about 7.5 to about 8.5, from about 8.0 to about 9.0, from about 8.5 to about 9.5, or from about 9.0 to about 10.0.

In some cases, compositions of the present disclosure are prepared according to good manufacturing practice (GMP) and/or current GMP (cGMP). Guidelines used for implementing GMP and/or cGMP may be obtained from one or more of the US Food and Drug Administration (FDA), the World Health Organization (WHO), and the International Conference on Harmonization (ICH).

### Dosage and administration

For treatment of human subjects, zilucoplan may be formulated as pharmaceutical compositions. Depending on the subject to be treated, the mode of administration, and the type of treatment desired (e.g., prevention, prophylaxis, or therapy) zilucoplan may be formulated in ways consonant with these parameters. A summary of such techniques is found in Remington: The Science and Practice of Pharmacy, 21st Edition, Lippincott Williams & Wilkins, (2005); and Encyclopedia of Pharmaceutical Technology, eds. J. Swarbrick and J. C. Boylan, 1988-1999, Marcel Dekker, New York.

Zilucoplan may be provided in a therapeutically effective amount. In some cases, a therapeutically effective amount of zilucoplan may be achieved by administration of a dose of from about 0.1 mg to about 1 mg, from about 0.5 mg to about 5 mg, from about 1 mg to about 20 mg, from about 5 mg to about 50 mg, from about 10 mg to about 100 mg, from about 20 mg to about 200 mg, or at least 200 mg of zilucoplan.

In some embodiments, subjects may be administered a therapeutic amount of a zilucoplan based on the weight of such subjects. In some cases, zilucoplan is administered at a dose of from about 0.001 mg/kg to about 1.0 mg/kg, from about 0.01 mg/kg to about 2.0 mg/kg, from about 0.05 mg/kg to about 5.0 mg/kg, from about 0.03 mg/kg to about 3.0 mg/kg, from about 0.01 mg/kg to about 10 mg/kg, from about 0.1 mg/kg to about 2.0 mg/kg, from about 0.2 mg/kg to about 3.0 mg/kg, from about 0.4 mg/kg to about 4.0 mg/kg, from about 1.0 mg/kg to about 5.0 mg/kg, from about 2.0 mg/kg to about 4.0 mg/kg, from about 1.5 mg/kg to about 7.5 mg/kg, from about 5.0 mg/kg to about 15 mg/kg, from about 7.5 mg/kg to about 12.5 mg/kg, from about 10 mg/kg to about 20 mg/kg, from about 15 mg/kg to about 30 mg/kg, from about 20 mg/kg to about 40 mg/kg, from about 30 mg/kg to about 60 mg/kg, from about 40 mg/kg to about 80 mg/kg, from about 50 mg/kg to about 100 mg/kg, or at least 100 mg/kg. Such ranges may include ranges suitable for administration to human subjects. Dosage levels may be highly dependent on the nature of the condition; drug efficacy; the condition of the patient; the judgment of the practitioner; and the frequency and mode of administration. In some embodiments, zilucoplan may be administered at a dose of from about 0.01 mg/kg to about 10 mg/kg. In some cases, zilucoplan may be administered at a dose of from about 0.1 mg/kg to about 3 mg/kg.

In some cases, zilucoplan are provided at concentrations adjusted to achieve a desired level of the C5 inhibitor in a sample, biological system, or subject (e.g., plasma level in a subject). In some cases, desired concentrations of zilucoplan in a sample, biological system, or subject may include concentrations of from about 0.001 µM to about 0.01 µM, from about 0.005 µM to about 0.05 µM, from about 0.02 µM to about 0.2 µM, from about 0.03 µM to about 0.3 µM, from about 0.05 µM to about 0.5 µM, from about 0.01 µM to about 2.0 µM, from about 0.1 µM to about 50 µM, from about 0.1 µM to about 10 µM, from about 0.1 µM to about 5 µM, from about 0.2 µM to about 20 µM, from about 5 µM to about 100 µM, or from about 15 µM to about 200 µM. In some cases, desired concentrations of zilucoplan in subject plasma may be from about 0.1 µg/mL to about 1000 µg/mL. The desired concentration of zilucoplan in subject plasma may be from about 0.01 µg/mL to about 2 µg/mL, from about 0.02 µg/mL to about 4 µg/mL, from about 0.05 µg/mL to about 5 µg/mL, from about 0.1 µg/mL to about 1.0 µg/mL, from about 0.2 µg/mL to about 2.0 µg/mL, from about 0.5 µg/mL to about 5 µg/mL, from about 1 µg/mL to about 5 µg/mL, from about 2 µg/mL to about 10 µg/mL, from about 3 µg/mL to about 9 µg/mL, from about 5 µg/mL to about 20 µg/mL, from about 10 µg/mL to about 40 µg/mL, from about 30 µg/mL to about 60 µg/mL, from about 40 µg/mL to about 80 µg/mL, from about 50 µg/mL to about 100 µg/mL, from about 75 µg/mL to about 150 µg/mL, or at least 150 µg/mL. In other embodiments, zilucoplan is administered at a dose sufficient to achieve a maximum serum concentration (Cₘₐₓ) of at least 0.1 µg/mL, at least 0.5 µg/mL, at least 1 µg/mL, at least 5 µg/mL, at least 10 µg/mL, at least 50 µg/mL, at least 100 µg/mL, or at least 1000 µg/mL.

In some embodiments, zilucoplan is administered daily at a dose sufficient to deliver from about 0.1 mg/day to about 60 mg/day per kg weight of a subject. In some cases, the Cₘₐₓ achieved with each dose is from about 0.1 µg/mL to about 1000 µg/mL. In such cases, the area under the curve (AUC) between doses may be from about 200 µg*hr/mL to about 10,000 µg*hr/mL.

According to some methods of the present disclosure, zilucoplan is provided at concentrations needed to achieve a desired effect. In some cases, compounds and compositions of the disclosure are provided at an amount necessary to reduce a given reaction or process by half. The concentration needed to achieve such a reduction is referred to herein as the half maximal inhibitory concentration, or "IC₅₀." Alternatively, compounds and compositions of the disclosure may be provided at an amount necessary to increase a given reaction, activity or process by half. The concentration needed for such an increase is referred to herein as the half maximal effective concentration or "EC₅₀."

Zilucoplan may be present in amounts totaling 0.1-95% by weight of the total weight of the composition. In some cases, zilucoplan is provided by intravenous (IV) administration. In some cases, zilucoplan is provided by subcutaneous (SC) administration.

SC administration of zilucoplan may, in some cases, provide advantages over IV administration. SC administration may allow patients to provide self-treatment. Such treatment may be advantageous in that patients could provide treatment to themselves in their own home, avoiding the need to travel to a provider or medical facility. Further, SC treatment may allow patients to avoid long-term complications associated with IV administration, such as infections, loss of venous access, local thrombosis, and hematomas. In some embodiments, SC treatment may increase patient compliance, patient satisfaction, quality of life, reduce treatment costs and/or drug requirements.

In some cases, daily SC administration provides steady-state zilucoplan concentrations that are reached within 1-3 doses, 2-3 doses, 3-5 doses, or 5-10 doses. In some cases, daily SC doses of from about 0.1 mg/kg to about 0.3 mg/kg may achieve sustained zilucoplan levels greater than or equal to 2.5 µg/mL and/or inhibition of complement activity of greater than 90%.

Zilucoplan may exhibit slow absorption kinetics (time to maximum observed concentration of greater than 4-8 hours) and high bioavailability (from about 75% to about 100%) after SC administration.

In some embodiments, dosage and/or administration are altered to modulate the half-life (t_{1/2}) of zilucoplan levels in a subject or in subject fluids (e.g., plasma). In some cases, t_{1/2} is at least 1 hour, at least 2 hrs, at least 4 hrs, at least 6 hrs, at least 8 hrs, at least 10 hrs, at least 12 hrs, at least 16 hrs, at least 20 hrs, at least 24 hrs, at least 36 hrs, at least 48 hrs, at least 60 hrs, at least 72 hrs, at least 96 hrs, at least 5 days, at least 6 days, at least 7 days, at least 8 days, at least 9 days, at least 10 days, at least 11 days, at least 12 days, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, or at least 16 weeks.

In some embodiments, zilucoplan may exhibit long terminal t_{1/2}. Extended terminal t_{1/2} may be due to extensive target binding and/or additional plasma protein binding. In some cases, zilucoplan exhibits t_{1/2} values greater than 24 hours in both plasma and whole blood. In some cases, zilucoplan does not lose functional activity after incubation in human whole blood at 37°C for 16 hours.

In some embodiments, dosage and/or administration are altered to modulate the steady state volume of distribution of zilucoplan. In some cases, the steady state volume of distribution of zilucoplan is from about 0.1 mL/kg to about 1 mL/kg, from about 0.5 mL/kg to about 5 mL/kg, from about 1 mL/kg to about 10 mL/kg, from about 5 mL/kg to about 20 mL/kg, from about 15 mL/kg to about 30 mL/kg, from about 10 mL/kg to about 200 mL/kg, from about 20 mL/kg to about 60 mL/kg, from about 30 mL/kg to about 70 mL/kg, from about 50 mL/kg to about 200 mL/kg, from about 100 mL/kg to about 500 mL/kg, or at least 500 mL/kg. In some cases, the dosage and/or administration of zilucoplan is adjusted to ensure that the steady state volume of distribution is equal to at least 50% of total blood volume. In some embodiments, zilucoplan distribution may be restricted to the plasma compartment.

In some embodiments, zilucoplan exhibits a total clearance rate of from about 0.001 mL/hr/kg to about 0.01 mL/hr/kg, from about 0.005 mL/hr/kg to about 0.05 mL/hr/kg, from about 0.01 mL/hr/kg to about 0.1 mL/hr/kg, from about 0.05 mL/hr/kg to about 0.5 mL/hr/kg, from about 0.1 mL/hr/kg to about 1 mL/hr/kg, from about 0.5 mL/hr/kg to about 5 mL/hr/kg, from about 0.04 mL/hr/kg to about 4 mL/hr/kg, from about 1 mL/hr/kg to about 10 mL/hr/kg, from about 5 mL/hr/kg to about 20 mL/hr/kg, from about 15 mL/hr/kg to about 30 mL/hr/kg, or at least 30 mL/hr/kg.

Time periods for which maximum concentration of zilucoplan in subjects (e.g., in subject serum) is maintained (Tₘₐₓ values) may be adjusted by altering dosage and/or administration (e.g., subcutaneous administration). In some cases, zilucoplan have Tₘₐₓ values of from about 1 min to about 10 min, from about 5 min to about 20 min, from about 15 min to about 45 min, from about 30 min to about 60 min, from about 45 min to about 90 min, from about 1 hour to about 48 hrs, from about 2 hrs to about 10 hrs, from about 5 hrs to about 20 hrs, from about 10 hrs to about 60 hrs, from about 1 day to about 4 days, from about 2 days to about 10 days, or at least 10 days.

In some embodiments, zilucoplan may be administered without off-target effects. In some cases, zilucoplan does not inhibit hERG (human ether-a-go-go related gene), even with concentrations less than or equal to 300 µM. SC injection of zilucoplan with dose levels up to 10 mg/kg may be well tolerated and not result in any adverse effects of the cardiovascular system (e.g., elevated risk of prolonged ventricular repolarization) and/or respiratory system.

Zilucoplan doses may be determined using the no observed adverse effect level (NOAEL) observed in another species. Such species may include, but are not limited to monkeys, rats, rabbits, and mice. In some cases, human equivalent doses (HEDs) may be determined by allometric scaling from NOAELs observed in other species. In some cases, HEDs result in therapeutic margins of from about 2 fold to about 5 fold, from about 4 fold to about 12 fold, from about 5 fold to about 15 fold, from about 10 fold to about 30 fold, or at least 30 fold. In some cases, therapeutic margins are determined by using exposure in primates and estimated human Cₘₐₓ levels in humans.

In some embodiments, zilucoplan allows for a rapid washout period in cases of infection where prolonged inhibition of the complement system is detrimental.

Zilucoplan administration according to the present disclosure may be modified to reduce potential clinical risks to subjects. Infection with *Neisseria meningitidis* is a known risk of C5 inhibitors, including eculizumab. In some cases, risk of infection with *Neisseria meningitides* is minimized by instituting one or more prophylactic steps. Such steps may include the exclusion of subjects who may already be colonized by these bacteria. In some cases, prophylactic steps may include coadministration with one or more antibiotics. In some cases, ciprofloxacin may be coadministered. In some cases, ciprofloxacin may be coadministered orally at a dose of from about 100 mg to about 1000 mg (e.g., 500 mg).

In some embodiments, zilucoplan is administered at a frequency of every hour, every 2 hrs, every 4 hrs, every 6 hrs, every 12 hrs, every 18 hrs, every 24 hrs, every 36 hrs, every 72 hrs, every 84 hrs, every 96 hrs, every 5 days, every 7 days, every 10 days, every 14 days, every week, every two weeks, every 3 weeks, every 4 weeks, every month, every 2 months, every 3 months, every 4 months, every 5 months, every 6 months, every year, or at least every year. In some cases, zilucoplan is administered once daily or administered as two, three, or more sub-doses at appropriate intervals throughout the day.

In some embodiments, zilucoplan is administered in multiple daily doses. In some cases, zilucoplan is administered daily for 7 days. In some cases, zilucoplan is administered daily for 7 to 100 days. In some cases, zilucoplan is administered daily for at least 100 days. In some cases, zilucoplan is administered daily for an indefinite period.

Zilucoplan delivered intravenously may be delivered by infusion over a period of time, such as over a 5 minute, 10 minute, 15 minute, 20 minute, or 25 minute period. The administration may be repeated, for example, on a regular basis, such as hourly, daily, weekly, biweekly (i.e., every two weeks), for one month, two months, three months, four months, or more than four months. After an initial treatment regimen, treatments may be administered on a less frequent basis. For example, after biweekly administration for three months, administration may be repeated once per month, for six months or a year or longer. Zilucoplan administration may reduce, lower, increase or alter binding or any physiologically deleterious process (e.g., in a cell, tissue, blood, urine or other compartment of a patient) by at least 10%, at least 15%, at least 20%, at least 25%, at least 30%, at least 40%, at least 50%, at least 60%, at least 70%, at least 80 % or at least 90% or more.

Before administration of a full dose of zilucoplan, patients can be administered a smaller dose, such as 5% of a full dose, and monitored for adverse effects, such as an allergic reaction or infusion reaction, or for elevated lipid levels or blood pressure. In another example, patients can be monitored for unwanted immunostimulatory effects, such as increased cytokine (*e.g*., TNF-alpha, IL-1, IL-6, or IL-10) levels.

Genetic predisposition plays a role in the development of some diseases or disorders. Therefore, patients in need of zilucoplan may be identified by family history analysis, or, for example, screening for one or more genetic markers or variants. Healthcare providers (e.g., doctors or nurses) or family members may analyze family history information before prescribing or administering therapeutic compositions of the present disclosure.

### III. Kits and Devices

Zilucoplan may be included in a kit or device. These kits and devices are for use in the treatment of ALS.

### Kits

In some embodiments, zilucoplan is provided as part of a kit.

Kit components may be packaged in liquid (e.g., aqueous or organic) media or in dry (e.g., lyophilized) form. Kits may include containers that may include, but are not limited to vials, test tubes, flasks, bottles, syringes, or bags. Kit containers may be used to aliquot, store, preserve, insulate, and/or protect kit components. Kit components may be packaged together or separately. Some kits may include containers of sterile, pharmaceutically acceptable buffer and/or other diluent (e.g., phosphate buffered saline). In some embodiments, kits include containers of kit components in dry form with separate containers of solution for dissolving dried components. In some embodiments, kits include a syringe for administering one or more kit components.

When zilucoplan is provided as a dried powder it is contemplated that between 10 micrograms and 1000 milligrams of zilucoplan, or at least or at most those amounts are provided in kits.

Containers may include at least one vial, test tube, flask, bottle, syringe and/or other receptacle, into which polypeptide formulations may be placed, preferably, suitably allocated. Kits may also include containers for sterile, pharmaceutically acceptable buffer and/or other diluent.

Kits may include instructions for employing kit components as well the use of any other reagent not included in the kit. Instructions may include variations that can be implemented.

In some embodiments, the kits include syringes with zilucoplan and instructions. Syringes may be self-injection devices. Self-injection devices may include BD ULTRASAFE PLUS^{™} self-administration devices (BD, Franklin Lakes, NJ). Kits may include one or more items for addressing syringe wounds. Such items may include, but are not limited to, alcohol wipes and wound dressings (e.g., cotton balls, mesh pads, bandages, tape, gauze, etc.). Kits may further include disposal containers for disposal of used kit components. Disposal containers may be designed for disposal of sharp objects, such as needles and syringes. Some kits may include instructions for sharp object disposal.

In some embodiments, kits include zilucoplan in powdered form or in solution. Solutions may be aqueous solutions. Solutions may include PBS. Zilucoplan solutions may include from about 4 mg/ml to about 200 mg/ml zilucoplan. In some embodiments, zilucoplan solutions include about 40 mg/ml zilucoplan. Zilucoplan solutions may include preservatives. In some embodiments, zilucoplan solutions are preservative-free.

### Devices

In some embodiments, zilucoplan may be provided as part of a device. Devices may include pre-loaded syringes. As used herein, a "pre-loaded syringe" refers to a delivery device for injection administration, wherein the device is manufactured, prepared, packaged, stored, and/or distributed with a payload to be injected that is included within the device. Due to cyclic peptide stability, cyclic peptide inhibitors are especially well suited for manufacture, storage, and distribution in pre-loaded syringes. Further, pre-loaded syringes are especially well suited for self-administration (i.e., administration by a subject, without the aid of a medical professional). Self-administration represents a convenient way for subjects to obtain treatments without relying on medical professionals who may be located at a distance or are otherwise difficult to access. This makes self-administration options well suited for treatments requiring frequent injections (e.g., daily injections).

The pre-loaded syringes may include zilucoplan compositions formulated for injection. The compositions may be formulated for subcutaneous injection. Zilucoplan may be included in pre-loaded syringes in a solution of phosphate buffered saline. Zilucoplan may be present in the solution at a concentration of from about 4 mg/ml to about 400 mg/ml. In some embodiments, pre-loaded syringes include a 40 mg/ml solution of zilucoplan in PBS. In some embodiments, the syringes may include a volume of from about 0.1 ml to about 1 ml or from about 0.5 ml to about 2 ml. The solution may include a preservative.

Pre-loaded syringes may include ULTRASAFE PLUS^{™} passive needle guards (Becton Dickenson, Franklin Lakes, NJ). Other pre-loaded syringes include injection pens. Injection pens may be multi-dose pens. Some pre-loaded syringes include a needle. In some embodiments, the needle gauge is from about 20 to about 34. The needle gauge may be from about 29 to about 31.

### IV. Definitions

*Administered in combination:* As used herein, the term "administered in combination" or "combined administration" means that a subject is simultaneously exposed to two or more agents administered at the same time or within an interval of time such that the subject is at some point in time simultaneously exposed to both and/or such that there may be an overlap in the effect of each agent on the patient. In some embodiments, at least one dose of one or more agents is administered within about 24 hours, 12 hours, 6 hours, 3 hours, 1 hour, 30 minutes, 15 minutes, 10 minutes, 5 minutes, or 1 minute of at least one dose of one or more other agents. In some embodiments, administration occurs in overlapping dosage regimens. As used herein, the term "dosage regimen" refers to a plurality of doses spaced apart in time. Such doses may occur at regular intervals or may include one or more hiatus in administration. *Bioavailability.* As used herein, the term "bioavailability" refers to the systemic availability of a given amount of a compound (e.g., C5 inhibitor) administered to a subject. Bioavailability can be assessed by measuring the area under the curve (AUC) or the maximum serum or plasma concentration (Cₘₐₓ) of the unchanged form of a compound following administration of the compound to a subject. AUC is a determination of the area under the curve when plotting the serum or plasma concentration of a compound along the ordinate (Y-axis) against time along the abscissa (X-axis). Generally, the AUC for a particular compound can be calculated using methods known to those of ordinary skill in the art and/or as described in G. S. Banker, Modern Pharmaceutics, Drugs and the Pharmaceutical Sciences, v. 72, Marcel Dekker, New York, Inc., 1996.

*Biological system:* As used herein, the term "biological system" refers to a cell, a group of cells, a tissue, an organ, a group of organs, an organelle, a biological fluid, a biological signaling pathway (e.g., a receptor-activated signaling pathway, a charge-activated signaling pathway, a metabolic pathway, a cellular signaling pathway, etc.), a group of proteins, a group of nucleic acids, or a group of molecules (including, but not limited to biomolecules) that carry out at least one biological function or biological task within cellular membranes, cellular compartments, cells, cell cultures, tissues, organs, organ systems, organisms, multicellular organisms, biological fluids, or any biological entities. In some embodiments, biological systems are cell signaling pathways that include intracellular and/or extracellular signaling biomolecules. In some embodiments, biological systems include proteolytic cascades (e.g., the complement cascade).

*Buffering agent:* As used herein, the term "buffering agent" refers to a compound used in a solution for the purposes of resisting changes in pH. Such compounds may include, but are not limited to acetic acid, adipic acid, sodium acetate, benzoic acid, citric acid, sodium benzoate, maleic acid, sodium phosphate, tartaric acid, lactic acid, potassium metaphosphate, glycine, sodium bicarbonate, potassium phosphate, sodium citrate, and sodium tartrate.

*Clearance rate:* As used herein, the term "clearance rate" refers to the velocity at which a particular compound is cleared from a biological system or fluid.

*Compound:* As used herein, the term "compound," refers to a distinct chemical entity. In some embodiments, a particular compound may exist in one or more isomeric or isotopic forms (including, but not limited to stereoisomers, geometric isomers and isotopes). In some embodiments, a compound is provided or utilized in only a single such form. In some embodiments, a compound is provided or utilized as a mixture of two or more such forms (including, but not limited to a racemic mixture of stereoisomers). Those of skill in the art will appreciate that some compounds exist in different forms, show different properties and/or activities (including, but not limited to biological activities). In such cases it is within the ordinary skill of those in the art to select or avoid particular forms of a compound for use in accordance with the present disclosure. For example, compounds that contain asymmetrically substituted carbon atoms can be isolated in optically active or racemic forms.

*Cyclic or Cyclized:* As used herein, the term "cyclic" refers to the presence of a continuous loop. The continuous loop may be formed by a chemical bond between different regions of a compound (also referred to herein as a "cyclic bond.") Cyclic molecules need not be circular, only joined to form an unbroken chain of subunits. Cyclic polypeptides may include a "cyclic loop," formed when two amino acids are connected by a bridging moiety. The cyclic loop comprises the amino acids along the polypeptide present between the bridged amino acids. Cyclic loops may include 2, 3, 4, 5, 6, 7, 8, 9, 10 or more amino acids.

*Downstream event:* As used herein, the term "downstream" or "downstream event," refers to any event occurring after and/or as a result of another event. In some cases, downstream events are events occurring after and as a result of C5 cleavage and/or complement activation. Such events may include, but are not limited to, generation of C5 cleavage products, activation of MAC, hemolysis, and hemolysis-related disease (e.g., PNH).

*Equilibrium dissociation constant:* As used herein, the term "equilibrium dissociation constant" or "K_{D}" refers to a value representing the tendency of two or more agents (e.g., two proteins) to reversibly separate. In some cases, K_{D} indicates a concentration of a primary agent at which half of the total levels of a secondary agent are associated with the primary agent.

*Half-life:* As used herein, the term "half-life" or "t_{1/2}" refers to the time it takes for a given process or compound concentration to reach half of a final value. The "terminal half-life" or "terminal t_{1/2}" refers to the time needed for the plasma concentration of a factor to be reduced by half after the concentration of the factor has reached a pseudo-equilibrium.

*Identity:* As used herein, the term "identity," when referring to polypeptides or nucleic acids, refers to a comparative relationship between sequences. The term is used to describe the degree of sequence relatedness between polymeric sequences and may include the percentage of matching monomeric components with gap alignments (if any) addressed by a particular mathematical model or computer program (i.e., "algorithms"). Identity of related polypeptides can be readily calculated by known methods. Such methods include, but are not limited to, those described previously by others (Lesk, A. M., ed., Computational Molecular Biology, Oxford University Press, New York, 1988; Smith, D. W., ed., Biocomputing: Informatics and Genome Projects, Academic Press, New York, 1993; Griffin, A. M. et al., ed., Computer Analysis of Sequence Data, Part 1, Humana Press, New Jersey, 1994; von Heinje, G., Sequence Analysis in Molecular Biology, Academic Press, 1987; Gribskov, M. et al., ed., Sequence Analysis Primer, M. Stockton Press, New York, 1991; and Carillo et al., Applied Math, SIAM J, 1988, 48, 1073).

*Inhibitor:* As used herein, the term "inhibitor" refers to any agent that blocks or causes a reduction in the occurrence of a specific event; cellular signal; chemical pathway; enzymatic reaction; cellular process; interaction between two or more entities; biological event; disease; disorder; or condition.

*Initial loading dose:* As used herein, an "initial loading dose" refers to a first dose of a therapeutic agent that may differ from one or more subsequent doses. Initial loading doses may be used to achieve an initial concentration of a therapeutic agent or level of activity before subsequent doses are administered.

*Intravenous:* As used herein, the term "intravenous" refers to the area within a blood vessel. Intravenous administration typically refers to delivery of a compound into the blood through injection in a blood vessel (e.g., vein).

*In vitro:* As used herein, the term *"in vitro"* refers to events that occur in an artificial environment (*e*.*g*., in a test tube or reaction vessel, in cell culture, in a Petri dish, *etc*.), rather than within an organism (*e*.*g*., animal, plant, or microbe).

*In vivo:* As used herein, the term *"in vivo"* refers to events that occur within an organism (*e*.*g*., animal, plant, or microbe or cell or tissue thereof).

*Lactam bridge:* As used herein, the term "lactam bridge" refers to an amide bond that forms a bridge between chemical groups in a molecule. In some cases, lactam bridges are formed between amino acids in a polypeptide.

*Linker:* The term "linker" as used herein refers to a group of atoms (*e*.*g*., 10-1,000 atoms), molecule(s), or other compounds used to join two or more entities. Linkers may join such entities through covalent or non-covalent (e.g., ionic or hydrophobic) interactions. Linkers may include chains of two or more polyethylene glycol (PEG) units. In some cases, linkers may be cleavable.

*Minute volume:* As used herein, the term "minute volume" refers to the volume of air inhaled or exhaled from a subject's lungs per minute.

*Non-proteinogenic:* As used herein, the term "non-proteinogenic" refers to any non-natural proteins, such as those with non-natural components, such as non-natural amino acids.

*Patient:* As used herein, "patient" refers to a subject who may seek or be in need of treatment, requires treatment, is receiving treatment, will receive treatment, or a subject who is under the care of a trained professional for a particular disease or condition.

*Pharmaceutical composition:* As used herein, the term "pharmaceutical composition" refers to a composition with at least one active ingredient (e.g., a C5 inhibitor) in a form and amount that permits the active ingredient to be therapeutically effective.

*Pharmaceutically acceptable:* The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

*Pharmaceutically acceptable excipients:* The phrase "pharmaceutically acceptable excipient," as used herein, refers to any ingredient other than active agents (e.g., active agent zilucoplan and/or active metabolites thereof or variants thereof) present in a pharmaceutical composition and having the properties of being substantially nontoxic and non-inflammatory in a patient. In some embodiments, a pharmaceutically acceptable excipient is a vehicle capable of suspending or dissolving the active agent. Excipients may include, for example: anti adherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspending or dispersing agents, sweeteners, and waters of hydration. Exemplary excipients include, but are not limited to: butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E, vitamin C, and xylitol.

*Plasma compartment:* As used herein, the term "plasma compartment" refers to intravascular space occupied by blood plasma.

*Salt:* As used herein, the term "salt" refers to a compound made up of a cation with a bound anion. Such compounds may include sodium chloride (NaCl) or other classes of salts including, but not limited to acetates, chlorides, carbonates, cyanides, nitrites, nitrates, sulfates, and phosphates. The term "salt" may also be used to refer to salt forms of polypeptides described herein (i.e., zilucoplan salt). Such polypeptide salts may include zilucoplan sodium salt.

*Sample:* As used herein, the term "sample" refers to an aliquot or portion taken from a source and/or provided for analysis or processing. In some embodiments, a sample is from a biological source (referred to herein as a "biological sample") such as a tissue, cell or component part (e.g., a body fluid, including but not limited to blood, mucus, lymphatic fluid, synovial fluid, cerebrospinal fluid, spinal fluid, saliva, amniotic fluid, amniotic cord blood, urine, vaginal fluid and semen). In some embodiments, a sample may be or include a homogenate, lysate or extract prepared from a whole organism or a subset of its tissues, cells or component parts, or a fraction or portion thereof, including but not limited to, for example, plasma, serum, spinal fluid, lymph fluid, the external sections of the skin, respiratory, intestinal, and genitourinary tracts, tears, saliva, milk, blood cells, tumors, or organs. In some embodiments, a sample is or includes a medium, such as a nutrient broth or gel, which may contain cellular components, such as proteins. In some embodiments, a "primary" sample is an aliquot of the source. In some embodiments, a primary sample is subjected to one or more processing (e.g., separation, purification, etc.) steps to prepare a sample for analysis or other use.

*Subcutaneous:* As used herein, the term "subcutaneous" refers to the space underneath the skin. Subcutaneous administration is delivery of a compound beneath the skin.

*Subject:* As used herein, the term "subject" refers to any organism to which a compound or method in accordance with the disclosure may be administered or applied, *e.g.,* for experimental, diagnostic, prophylactic, and/or therapeutic purposes. Typical subjects include animals (*e*.*g*., mammals such as mice, rats, rabbits, porcine subjects, non-human primates, and humans).

*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Therapeutically effective amount:* As used herein, the term "therapeutically effective amount" means an amount of an agent to be delivered (*e*.*g*., C5 inhibitor) that is sufficient, when administered to a subject suffering from or susceptible to a disease, disorder, and/or condition, to treat, improve symptoms of, diagnose, prevent, and/or delay the onset of the disease, disorder, and/or condition.

*Tidal volume:* As used herein, the term "tidal volume" refers to the normal lung volume of air displaced between breaths (in the absence of any extra effort).

*Tₘₐₓ:* As used herein, the term "Tₘₐₓ" refers to the time period for which maximum concentration of a compound in a subject or fluid is maintained.

*Treating:* As used herein, the term "treating" refers to partially or completely alleviating, ameliorating, improving, relieving, delaying onset of, inhibiting progression of, reducing severity of, and/or reducing incidence of one or more symptoms or features of a particular disease, disorder, and/or condition. Treatment may be administered to a subject who does not exhibit signs of a disease, disorder, and/or condition and/or to a subject who exhibits only early signs of a disease, disorder, and/or condition for the purpose of decreasing the risk of developing pathology associated with the disease, disorder, and/or condition.

*Treatment dose:* As used herein, "treatment dose" refers to one or more doses of a therapeutic agent administered in the course of addressing or alleviating a therapeutic indication. Treatment doses may be adjusted to maintain a desired concentration or level of activity of a therapeutic agent in a body fluid or biological system.

*Volume of distribution:* As used herein, the term "volume of distribution" or "V_{dist}" refers to a fluid volume required to contain the total amount of a compound in the body at the same concentration as in the blood or plasma. The volume of distribution may reflect the extent to which a compound is present in the extravascular tissue. A large volume of distribution reflects the tendency of a compound to bind to tissue components compared with plasma protein components. In a clinical setting, V_{dist} can be used to determine a loading dose of a compound to achieve a steady state concentration of that compound.

In the claims, articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or the entire group members are present in, employed in, or otherwise relevant to a given product or process.

It is also noted that the term "comprising" is intended to be open and permits but does not require the inclusion of additional elements or steps. When the term "comprising" is used herein, the term "consisting of" is thus also encompassed and disclosed.

Where ranges are given, endpoints are included. Furthermore, it is to be understood that unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise.

Section and table headings are not intended to be limiting.

### EXAMPLES

### Example 1. Preparation of zilucoplan aqueous solution

Polypeptides were synthesized using standard solid-phase Fmoc/tBu methods. The synthesis was performed on a Liberty automated microwave peptide synthesizer (CEM, Matthews NC) using standard protocols with Rink amide resin, although other automated synthesizers without microwave capability may also be used. All amino acids were obtained from commercial sources. The coupling reagent used was 2-(6-chloro-1-H-benzotriazolelyl)-1,1,3,3,-tetramethylaminium hexafluorophosphate (HCTU) and the base was diisopropylethylamine (DIEA). Polypeptides were cleaved from resin with 95% TFA, 2.5% TIS and 2.5% water for 3 hours and isolated by precipitation with ether. The crude polypeptides were purified on a reverse phase preparative HPLC using a C18 column, with an acetonitrile/water 0.1% TFA gradient from 20%-50% over 30 min. Fractions containing pure polypeptides were collected and lyophilized and all polypeptides were analyzed by LC-MS.

Zilucoplan (SEQ ID NO: 1), as described in International Publication Numbers WO2017105939 and WO2018106859 (see also CAS Number 1841136-73-9), was prepared as a cyclic peptide containing 15 amino acids (4 of which are non-natural amino acids), an acetylated N-terminus, and a C-terminal carboxylic acid. The C-terminal lysine of the core peptide has a modified side chain, forming a N-**ε**-(PEG24-**γ**-glutamic acid-N-α-hexadecanoyl) lysine reside. This modified side chain includes a polyethyleneglycol spacer (PEG24) attached to an L-γ glutamic acid residue that is derivatized with a palmitoyl group. The cyclization of zilucoplan is via a lactam bridge between the side-chains of L-Lys1 and L-Asp6. All of the amino acids in zilucoplan are L-amino acids. Zilucoplan has a molecular weight of 3562.23 g/mol and a chemical formula of C₁₇₂H₂₇₈N₂₄O₅₅.

Like eculizumab, zilucoplan blocks the proteolytic cleavage of C5 into C5a and C5b. Unlike eculizumab, zilucoplan can also bind to C5b and block C6 binding which prevents the subsequent assembly of the MAC.

Zilucoplan was prepared as an aqueous solution for injection containing 40 mg/mL of zilucoplan in a formulation of 50 mM sodium phosphate and 75.7 mM sodium chloride at a pH of 7.0. The resulting composition was used to prepare a medicinal product, in accordance with current Good Manufacturing Practices (cGMPs), the medicinal product including a 1 ml glass syringe with a 29 gauge, ½ inch staked needle placed within a needle safety self-administration device (ULTRASAFE PLUS^{™}, Becton Dickenson, Franklin Lakes, NJ) with finger flange and plunger rod.

### Example 2. Zilucoplan administration and storage

Zilucoplan is administered by subcutaneous (SC) or intravenous (IV) injection and the dose administered (dose volume) is adjusted based on subject weight on a mg/kg basis. This is achieved using a set of fixed doses aligned to a set of weight brackets. In total, human dosing supports a broad weight range of 43 to 109 kg (see Table 2). Injection volumes are approximately 0.4 mL to 0.8 mL. Subjects who present with a higher body weight (>109 kg) are accommodated on a case-by-case basis, in consultation with a medical monitor.

**Table 2. Dosage presentation by weight group**

| **Target dose (mg/kg)** | **Dose presentation** | | **Weight range (kg)** | **Dose range (mg/kg)** |
|---|---|---|---|---|
| | **Fill volume (mL)** | **Dose (mg)** | | |
| 0.3 | 0.416 | 16.6 | ≥ 43 to < 56 | 0.30 to 0.39 |
| 0.3 | 0.574 | 23 | ≥ 56 to < 77 | 0.30 to 0.41 |
| 0.3 | 0.810 | 32.4 | ≥ 77 to < 109 | 0.30 to 0.42 |

Zilucoplan is stored at 2°C to 8°C [36°F to 46°F]. Once dispensed to subjects, zilucoplan is stored at controlled room temperature (20°C to 25°C [68°F to 77°F]) for up to 30 days and is protected from sources of excessive temperature fluctuations such as high heat or exposure to light. Storage of zilucoplan outside of room temperatures is preferably avoided. Zilucoplan may be stored for up to 30 days under these conditions.

### Example 3. Zilucoplan treatment of ALS

ALS treatment with zilucoplan is carried out as part of a randomized, double-blind, and placebo-controlled study, followed by an open label, long-term extension. Study participants are selected from a broad patient population of individuals diagnosed with ALS that includes individuals from both familial and sporadic ALS patient populations with ALS diagnosed as possible, probable, lab-supported probable, or definite ALS (according to revised El Escorial criteria, see Ludolph, A. et al., ALS and FD, 2015. Early Online: 1-2), with less than or equal to three years since weakness onset, greater than fifty percent predicted vital capacity (based on sex, age, and height), and absent or stable dosing of riluzole or edaravone (at least one or more treatment cycle) for greater than or equal to 30 days at the time of screening.

Zilucoplan and matching placebo are supplied as sterile, preservative-free, aqueous solutions prefilled into 1 mL glass syringes (BD ULTRASAFE PLUS^{™} self-administration device, BD, Franklin Lakes, NJ) with 29 gauge, ½ inch, staked needles placed within self-administration devices. Fill volumes are adjusted based on subject weight range to achieve correct mg/kg dose range. Subjects are instructed to self-administer SC doses daily.

During the Treatment Period, subjects return to the clinic at predetermined intervals to evaluate safety, tolerability, and efficacy. Primary endpoint of the study is change in ALS Functional Rating Scale-Revised (ALSFRS-R) score from baseline through 24 weeks of treatment. Plasma and CSF concentrations of C5, zilucoplan, and any zilucoplan metabolites are assessed from samples taken during the study. Samples are also tested for complement activity by hemolysis assays and assays for detection of C5 cleavage products.

Treatment efficacy is also determined using various assessments carried out during clinical visits. These include slow vital capacity (SVC) assessment, muscle strength assessment by hand held dynamometry (HHD), speech capability assessment (including assessment of one or more of articulatory precision, speaking rate, percent of speech signal voiced, phonation length, consonant length, and nasality), and ALS biomarker profile assessment [including assessment of levels of one or more of neurofilament heavy chain (pNfH), neurofilament light chain (NfL), neurotrophin receptor p75 extracellular domain (p75ECD), soluble C5b-9, and C5 cleavage products]. Some efficacy determinations include home-based self-assessments that may be carried out with the use of a smart device (watch, mobile phone, computer, etc.).

Other laboratory assessments are also carried out during clinical visits. These include hematology panel assessments (hematocrit, hemoglobin, platelet count, red blood cells indices, total red blood cells, total white blood cells, and white blood cell & differential); blood chemistry panel/liver function tests (alanine aminotransferase, aspartate aminotransferase, albumin, alkaline phosphatase, bicarbonate, blood urea nitrogen, calcium, chloride, creatinine, glucose, magnesium, phosphate, potassium, sodium, total bilirubin and total protein, and uric acid); and urinalysis (albumin, bilirubin, blood, clarity, color, glucose, ketones, nitrate, pH, protein, specific gravity, urobilinogen, and white blood cell screen). Standard 12-lead ECG is also performed for review of tracings obtained. Physical and neurological examinations are performed to examine head/neck, eyes, ears, nose/throat, cardiovascular system, lungs, abdomen, musculoskeletal system, central nervous system, extremities, and skin.

Subjects treated with zilucoplan exhibit reduced or stabilized disease severity.

### Example 4. Direct assessment of complement C5 activity in CSF

Cerebrospinal fluid (CSF) samples obtained from human subjects treated with or without zilucoplan are assayed for complement activity and related biomarkers. C5 activity levels in CSF samples are assessed by combining CSF samples with C5-depleted normal human serum and testing for ability to promote red blood cell lysis (based on CSF-derived C5). CSF samples are also analyzed for zilucoplan levels, C5 levels, and levels of C5 activity biomarkers [e.g., soluble C5b-9 (sC5b-9)]. Samples from zilucoplan-treated subjects exhibited lower hemolysis activity levels and lower levels of C5 activity biomarkers with levels correlating with treatment-dependent zilucoplan inhibitor levels.

### Example 5. Central nervous system complement synthesis

C5-deficient mice were transplanted with human liver to enable expression of human C5. At a subsequent time point, mice were euthanized and serum and brain tissue samples were analyzed for human C5 levels. Human C5 was detected in mouse serum, but not in brain tissue. These results indicate that C5 does not cross the blood brain barrier (BBB) and that expression in brain is due to local synthesis.

### Example 6. Complement activity biomarker analysis

CSF and matching serum samples from human subjects with ALS (n=20) or without ALS (n=14) were assessed for levels of C5 and complement activity biomarkers. C5 levels were assessed by enzyme-linked immunosorbent assay (ELISA) using a commercial kit (catalog number ab125963; Abcam, Cambridge, UK) according to manufacturer instructions. Complement activity biomarkers were assessed by Quidel Complement Multiplex LLOQ assay (Quidel Corporation, San Diego, CA). Results are presented in Table 3. In the Table, p-values represent level of significance over corresponding healthy control levels (as determined by Mann-Whitney test).

**Table 3. Biomarker levels**

| **Biomarkers** | **Healthy Control CSF (ng/mL)** | **ALS CSF (ng/mL)** | **Significance (P-value)** | **Healthy Control Serum (ng/mL)** | **ALS Serum (ng/mL)** | **Significance (P-value)** |
|---|---|---|---|---|---|---|
| C5 | 153.60 | 142.75 | 0.7282 | 86140 | 88700 | 0.5327 |
| C5a | 0.04 | 0.41 | 0.0087 | 10.46 | 23.06 | 0.0001 |
| sC5b-9 | 3.98 | 25.95 | 0.0006 | 340.1 | 2393 | <0.0001 |
| C3a | 2.49 | 4.15 | 0.0650 | 787.0 | 1470 | 0.0081 |
| C4a | 25.40 | 25.42 | 0.9935 | 410.7 | 1187 | 0.0003 |
| Ba | 7.69 | 12.48 | 0.1232 | 760.9 | 1065 | 0.0007 |
| Bb | 4.64 | 9.77 | 0.0283 | 1374 | 1843 | 0.0105 |
| Factor I (fI) | 177.10 | 226.60 | 0.2075 | 259700 | 197100 | 0.006 |
| Factor H (fH) | 2036.00 | 2065.00 | 0.8575 | 234100 | 219900 | 0.4069 |

Levels of complement activity biomarkers C5a, sC5b-9, and Bb were significantly elevated in ALS CSF samples in comparison to samples from healthy controls. Levels of all complement activity biomarkers tested were significantly elevated in serum samples from subjects with ALS. Interestingly, no statistically significant differences in C5 CSF or serum levels were observed between healthy subjects and subjects with ALS, indicating that C5 is being replenished in CSF and serum compartments upon consumption. CSF levels of C5 were about 600 times lower than serum levels. Overall, these results indicate that complement activity biomarkers in subject serum and C5a, sC5b-9, and Bb levels in subject CSF correlate with ALS.

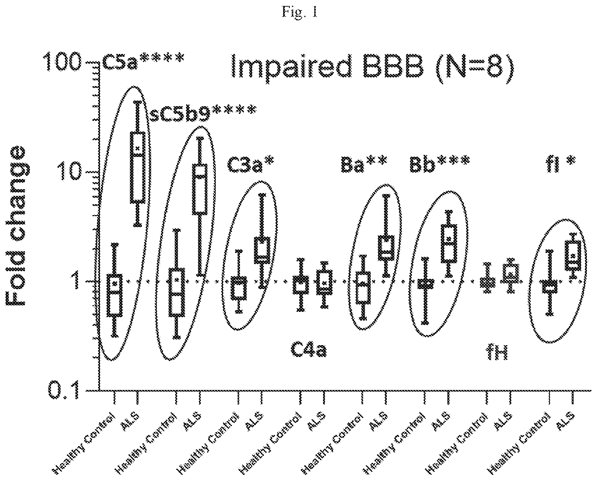

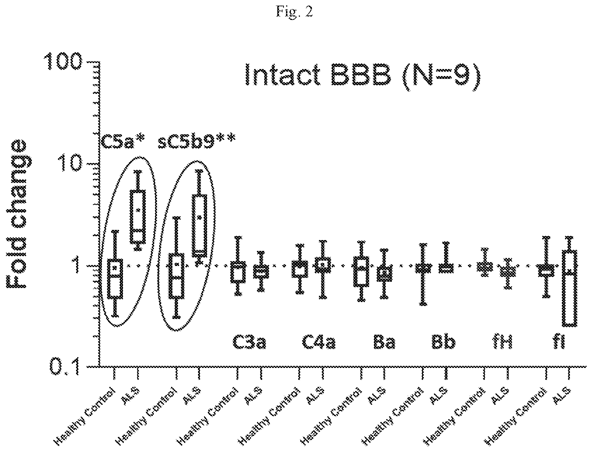

The ratio of CSF albumin levels to plasma albumin levels for each subject was calculated to obtain albumin-quotient values as a measure of BBB permeability. Albumin-quotient values indicated BBB impairment (greater than 9.0) for 47% of subjects with ALS and only 7% for healthy subjects. CSF biomarker levels were compared between subjects with impaired BBB (Fig. 1) and intact BBB (Fig. 2) based on albumin-quotient determination to evaluate complement activity and predominant complement activation pathway (classical, alternative, or lectin pathway). Markers of the alternative activation pathway (C3a, Ba and Bb) were significantly elevated in CSF of subjects with impaired BBB (Fig. 1). Interestingly, only markers of classical or lectin activation pathway, but not alternative pathway, were observed with similar biomarker analysis in corresponding plasma.

### Example 7. Subpopulation analysis

Complement activity biomarker analysis was conducted with CSF and matching plasma samples from human subjects with probable ALS and results were compared with those from subjects with definite ALS and healthy controls (as described in Example 6, above). Subjects with "definite" or "probable" ALS were identified according to revised El Escorial criteria as described in Brooks RB, et al. Amyotroph Lateral Scler Other Motor Neuron Disord. 2000 Dec; 1(5):293-9. Complete details related to subjects from which samples were obtained and analyzed are presented in Table 4.

**Table 4. Population characteristics**

| **Characteristic** | | **Subjects with definite or probable ALS: CSF: n=28, plasma: n=25** | **Healthy control subjects: n=14** |
|---|---|---|---|
| Gender: female/male (ratio) | | 8/20 (0.4) | 7/7 (1) |
| Age at biospecimen sampling: median (range) | | 65 (41-80) | 65 (54-70) |
| Symptom duration in months at sampling: median (range) | | 15.5 (2-56) | |
| Inheritance: familial/sporadic (ratio) | | 2/26 (0.077) | |
| Clinical presentation (revised El Escorial criteria): | | | |
| | Definite | 21 | |
| | Probable | 7 | |
| ALSFRS-R score | | | |
| | Score 40-48, mild to minimal | 18 | |
| | Score 30-39, moderate to mild | 9 | |
| | Score 21-29, severe to moderate | 1 | |

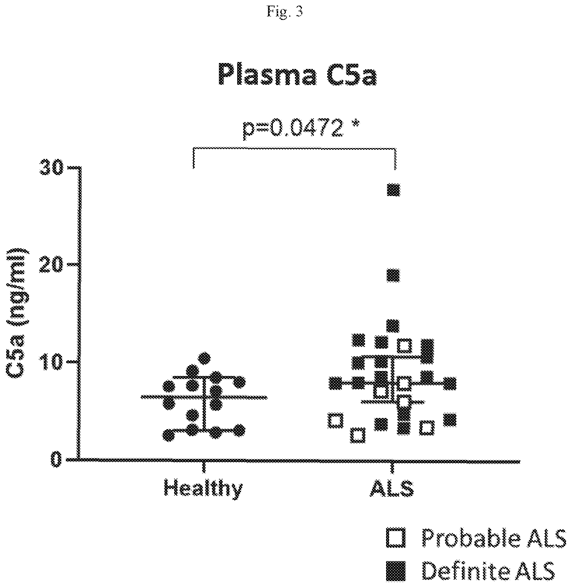

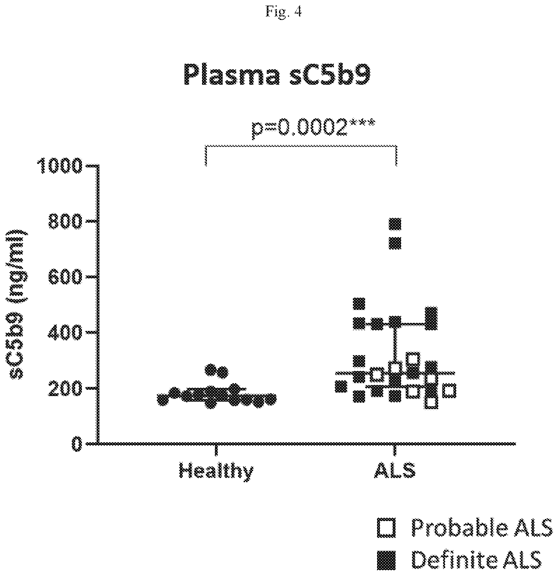

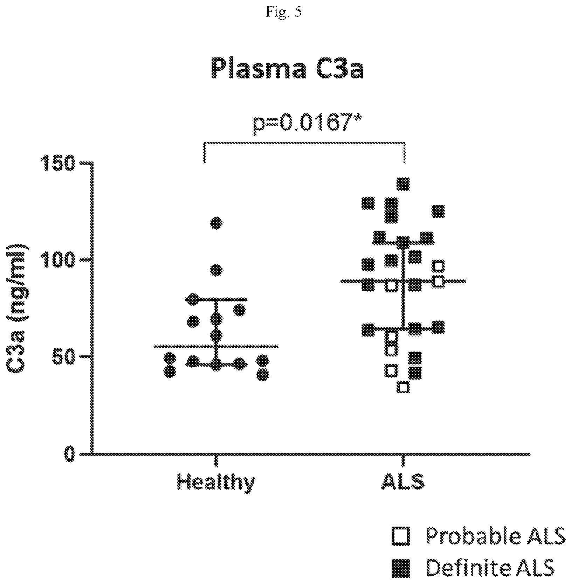

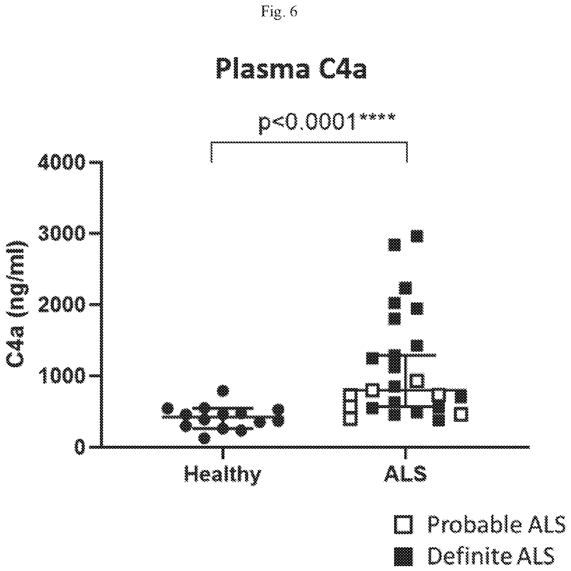

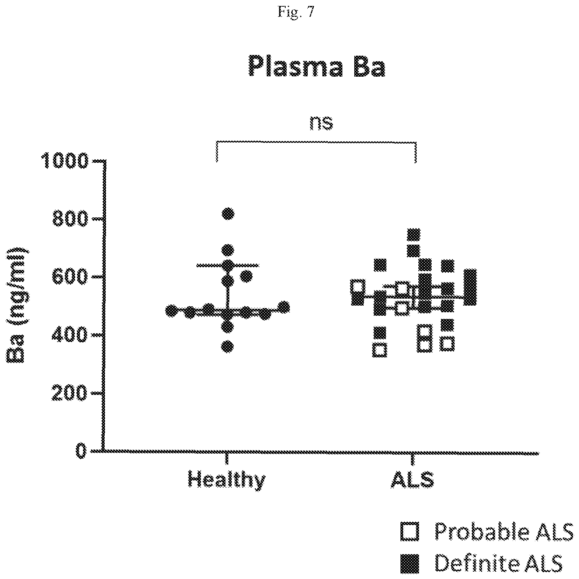

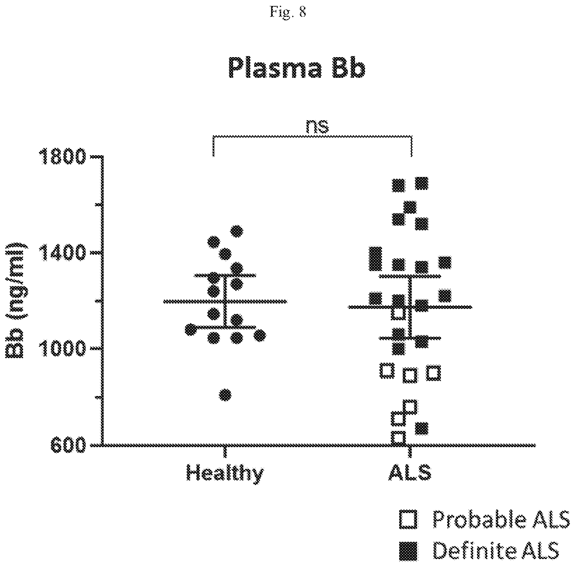

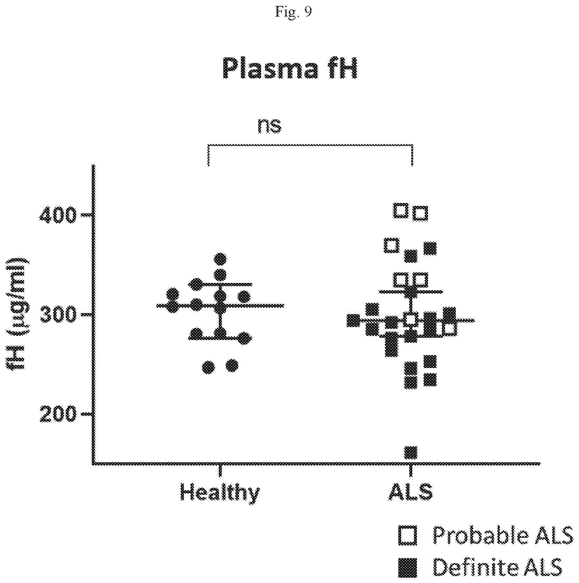

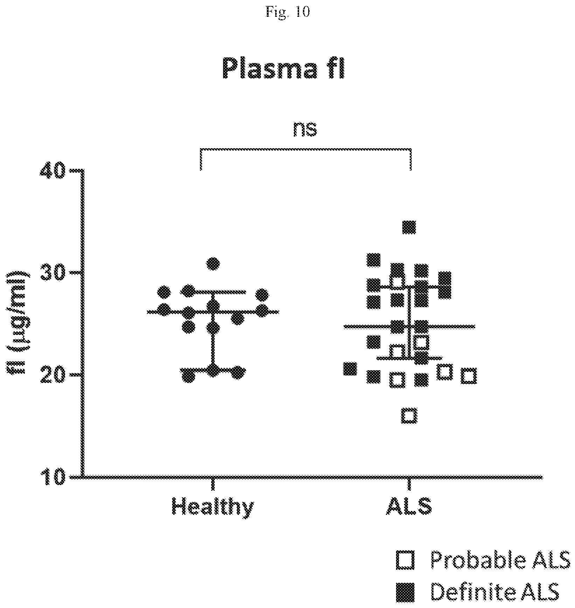

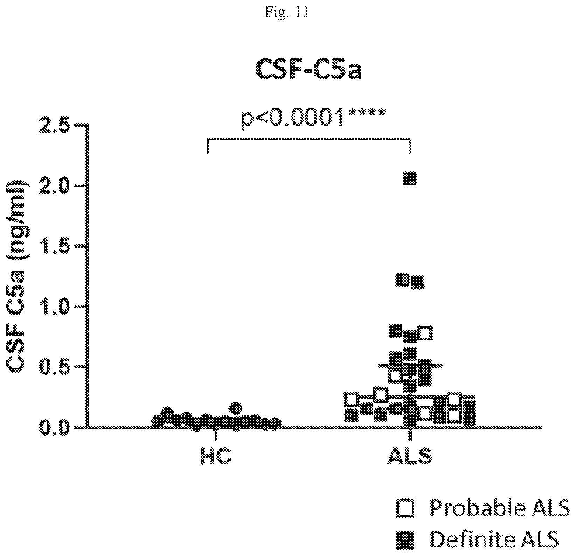

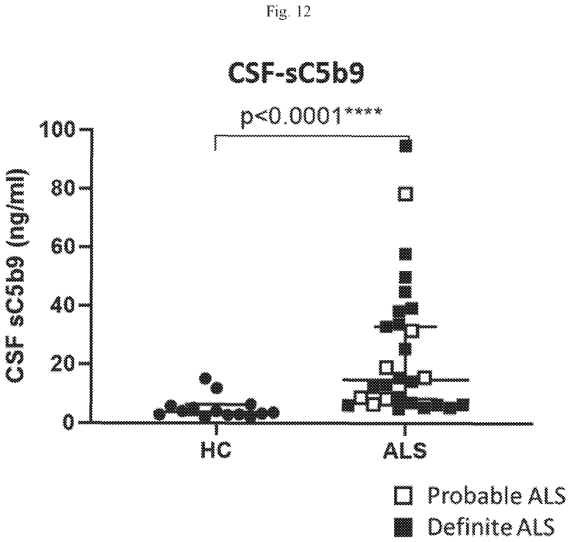

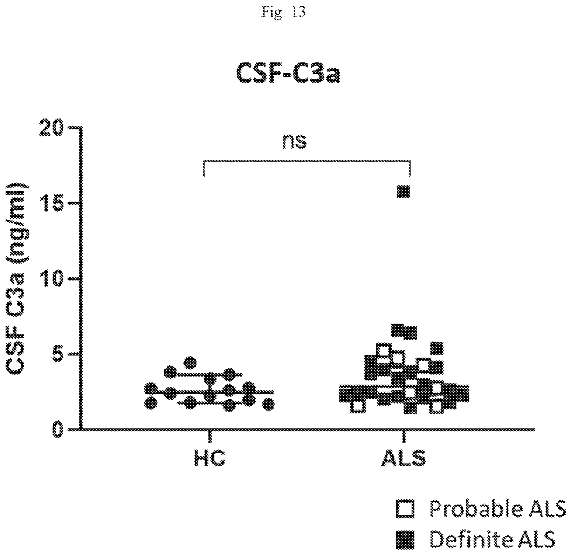

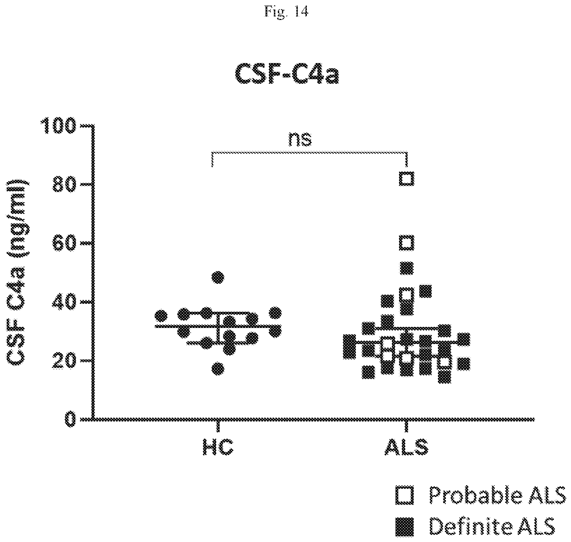

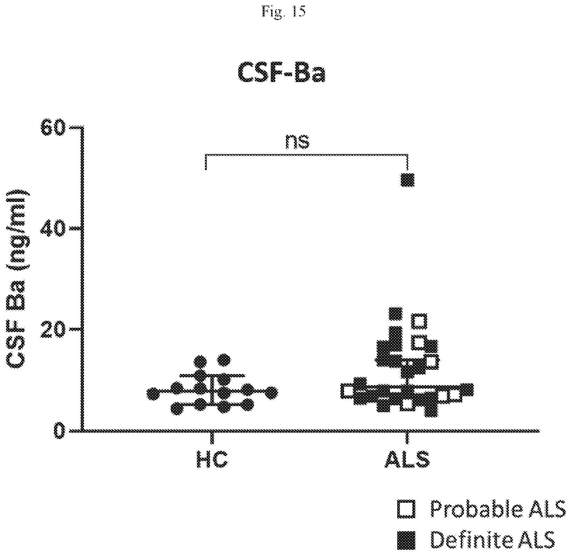

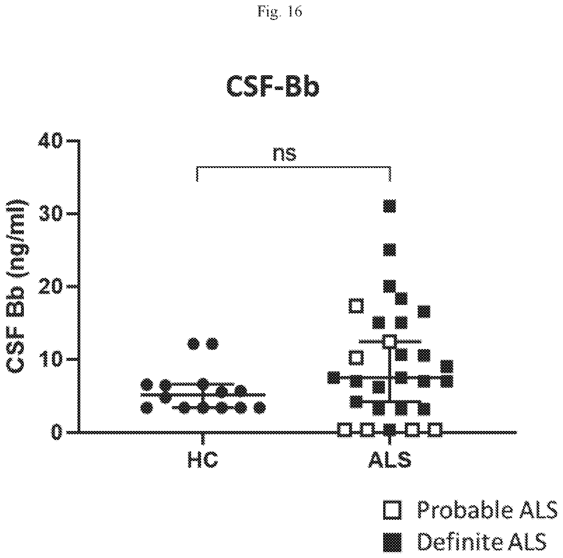

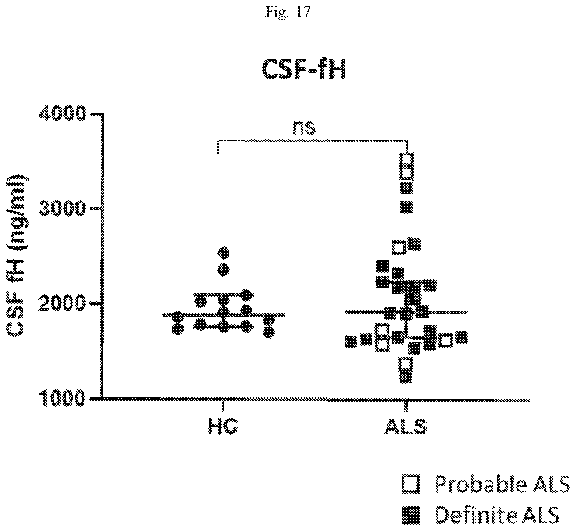

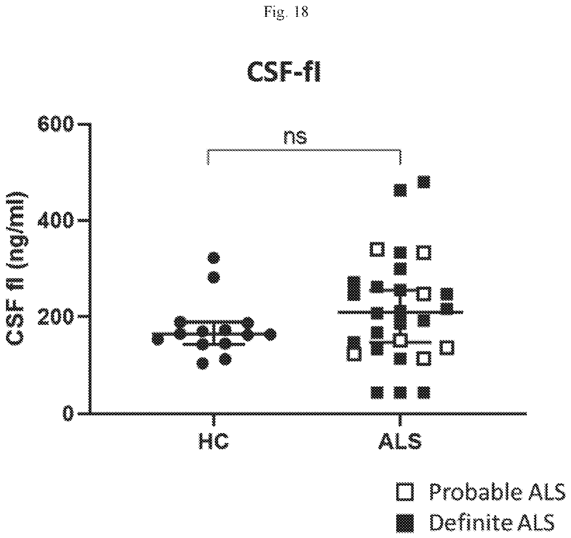

In plasma samples analyzed, C5a (Fig. 3), sC5b9 (Fig. 4), C3a (Fig. 5), and C4a (Fig. 6) levels were significantly elevated in subjects with ALS (definite and probable combined) as compared to healthy controls. Differences in Ba (Fig. 7), Bb (Fig. 8), fH (Fig. 9), and fl (Fig. 10) biomarker levels were not significant between healthy control plasma and plasma from subjects ASL (definite and probable ALS combined). In CSF, only C5a (Fig. 11) and sC5b9 (Fig. 12) levels were significantly elevated with definite or probable ALS as compared to CSF from healthy controls, while C3a (Fig. 13), C4a (Fig. 14), Ba (Fig. 15), Bb (Fig. 16), fH (Fig. 17), and fl (Fig. 18) biomarker levels were not.

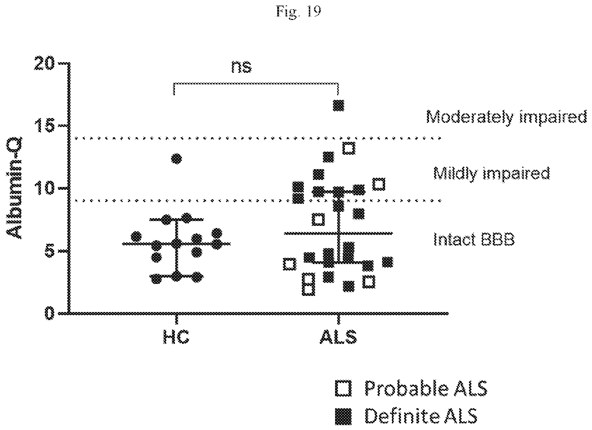

Albumin quotient values were also compared between subjects with definite or probable ALS and healthy control subjects (see Fig. 19). 36% of subjects with definite or probable ALS were determined to have blood brain barrier impairment compared to only 7% of healthy controls.

Overall, C5a and sC5b-9 levels produced in the CSF did not correlate with those observed in plasma. This finding, when taken together with observed elevation in complement split products in CSF in the absence of BBB impairment, supports local CNS complement activity in ALS that is independent from activity in the periphery.

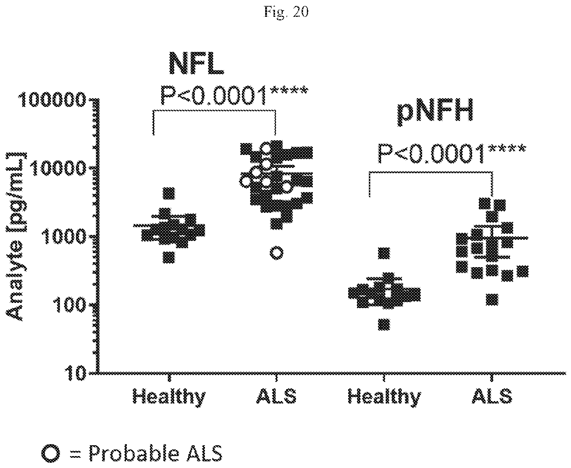

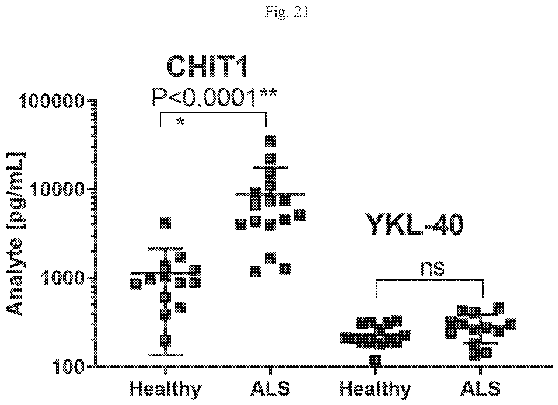

Levels of biomarkers associated with neuroaxonal degeneration (NfL and pNfH) and neuroinflammation [chitotriosidase-1 (CHIT1) and chitinase-3-like protein 1 (YKL-40)] were also analyzed in CSF samples from healthy control subjects and subjects with probable or definite ALS. Both NfL and pNfH levels were elevated in CSF from subjects with probable or definite ALS as compared to healthy control (Fig. 20). Levels of CHIT1 were also elevated in CSF samples from subjects with ALS as compared to healthy controls, while no statistical difference was observed in YKL-40 levels (Fig. 21).

### Example 8. Expanded subpopulation analysis

Complement activity biomarker analysis described in the previous Example was repeated with additional CSF and matching plasma samples from healthy human control subjects, human subjects with probable ALS, or human subjects with definite ALS. Subjects with "definite" or "probable" ALS were identified according to revised El Escorial criteria as described in Brooks RB, et al. Amyotroph Lateral Scler Other Motor Neuron Disord. 2000 Dec; 1(5):293-9. In the expanded analysis, subjects with "probable" ALS included: (1) subjects diagnosed based on presence of upper motor neuron (UMN) and lower motor neuron (LMN) signs in at least two regions with UMN signs rostral to LMN signs; and (2) subjects diagnosed with laboratory results-supported probable ALS based on presence of UMN and LMN signs in one region with evidence by electromyography (EMG) of LMN involvement in another region. Complete details related to subjects from which samples were obtained and analyzed are presented in Table 5.

**Table 5. Population characteristics**

| **Characteristic** | | **Subjects with definite or probable ALS (N=37): CSF: n=36, plasma: n=35** | **Healthy control subjects: n=17** |
|---|---|---|---|
| Gender: female/male (ratio) | | 9/28 (0.32) | 8/9 (0.89) |
| Age at biospecimen sampling: median (range) | | 65 (41-81) | 64 (55-70) |
| Symptom duration in months at sampling: median (range) | | 16.4 (2.1-55.3) | |
| Inheritance: familial/sporadic (ratio) | | 2/35 (0.057) | |
| Clinical presentation (revised El Escorial criteria): | | | |
| | Definite | 23 | |
| | Probable (including laboratory-supported probable ALS) | 14 | |
| ALSFRS-R score | | | |
| | Score 40-48, mild to minimal | 22 | |
| | Score 30-39, moderate to mild | 13 | |
| | Score 21-29, severe to moderate | 2 | |

Average biomarker levels detected in each subgroup are shown in Table 6. In the Table, p-values represent level of significance over corresponding healthy control levels (as determined by Kruskal-Wallis test).

**Table 6. Biomarker levels**

| **Biomarkers** | **Healthy Control CSF ng/ml** | **Probable ALS CSF ng/ml [p-value]** | **Definite ALS CSF ng/ml [p-value]** | **Healthy Control Plasma ng/ml** | **Probable ALS Plasma ng/ml [p-value]** | **Definite ALS Plasma ng/ml [p-value]** |
|---|---|---|---|---|---|---|
| C5a | 0.05 | 0.30 [0.0006] | 0.35 [<0.0001] | 7.70 | 6.71 [n.s.] | 10.10 [n.s.] |
| sC5b-9 | 5.31 | 13.00 [0.0067] | 14.18 [0.0007] | 176.00 | 161.10 [n.s.] | 287.90 [0.0013] |
| C3a | 2.42 | 4.17 [0.0212] | 3.41 [n.s.] | 64.32 | 64.33 [n.s.] | 108.40 [0.0004] |
| C4a | 30.42 | 41.00 [n.s.] | 26.83 [n.s.] | 387.50 | 648.40 [n.s.] | 1124.00 [<0.0001] |
| Ba | 8.25 | 11.68 [n.s.] | 9.70 [n.s.] | 489.00 | 446.20 [n.s.] | 537.00 [n.s.] |
| Bb | 6.50 | 12.00 [n.s.] | 10.00 [n.s.] | 1145.00 | 817.50 [0.0034] | 1180.00 [n.s.] |
| fH | 1828.00 | 2100.00 [n.s.] | 1875.00 [n.s.] | 309000.00 | 335000.00 [n.s.] | 284500.00 [n.s.] |
| fI | 161.80 | 229.50 [0.0029] | 203.70 [n.s.] | 26170.00 | 20300.00 [n.s.] | 27290.00 [n.s.] |

No significant differences in C5 levels were detected between any healthy control or ALS sample group. In plasma samples analyzed, sC5b9, C3a, and C4a levels were significantly elevated in subjects with definite ALS as compared to healthy controls, but not in subjects with probable ALS. In samples from probable ALS subjects, only Bb levels demonstrated a significant difference from healthy control levels. C5a and sC5b9 levels were significantly elevated in CSF samples from subjects with definite or probable ALS as compared to healthy controls. C3a and fl levels were significantly elevated only with probable ALS as compared to CSF from healthy controls.

As observed previously, C5a and sC5b-9 levels produced in the CSF did not correlate with those observed in plasma, further indicating local CNS complement activity in ALS that is independent from activity in the periphery.

In the expanded sample set, 29% of subjects with definite or probable ALS were determined to have BBB impairment (albumin-quotient value >9.0). Average biomarker levels detected in ALS CSF samples (definite and probable) associated with intact (N=26) or impaired (N=10) BBB are shown in Table 7 along with levels from healthy control CSF samples. In the Table, p-values represent level of significance over corresponding healthy control levels (as determined by Kruskal-Wallis test).

**Table 7. Biomarker levels**

| **Biomarkers** | **Healthy Control CSF ng/ml** | **BBB-intact ALS CSF ng/ml [p-value]** | **BBB-impaired ALS CSF ng/ml [p-value]** |
|---|---|---|---|
| C5 | 122.50 | 91.00 [n.s.] | 202.50 [n.s.] |
| C5a | 0.05 | 0.20 [0.0022] | 0.70 [<0.0001] |
| sC5b-9 | 5.31 | 12.29 [0.0131] | 42.68 [<0.0001] |
| C3a | 2.42 | 2.83 [n.s.] | 4.35 [0.0019] |
| C4a | 30.42 | 28.75 [n.s.] | 28.82 [n.s.] |
| Ba | 8.25 | 8.37 [n.s.] | 16.40 [0.0003] |
| Bb | 6.50 | 7.75 [n.s.] | 16.25 [0.0003] |
| fH | 1828.00 | 1803.00 [n.s.] | 2310.00 [0.0269] |
| fI | 161.80 | 197.30 [n.s.] | 263.20 [0.0001] |

Levels of C5a and sC5b-9 were significantly elevated in ALS CFS associated with both intact and impaired BBB, while C3a, Ba, Bb, fH, and fl were only significantly elevated in ALS CSF associated with impaired BBB.

## Claims

1. Zilucoplan for use in a method of treating amyotrophic lateral sclerosis (ALS) in a subject, the method comprising administering zilucoplan to the subject.

2. Zilucoplan for use of claim 1, wherein prior to treatment subject ALS disease severity is elevated and/or increasing at a specific rate; and
wherein treatment with zilucoplan stabilizes or reduces subject ALS disease severity, and/or reduces the specific rate at which subject ALS disease severity is increasing.

3. Zilucoplan for use of claim 2, wherein change in subject ALS disease severity is measured by ALS Functional Rating Scale-Revised (ALSFRS-R).

4. Zilucoplan for use of any one of claims 1-3, wherein prior to treatment, subject respiratory function is decreased and/or is declining at a specific rate; and wherein treatment with zilucoplan stabilizes or improves subject respiratory function and/or reduces the specific rate at which subject respiratory function is declining, wherein, optionally, change in subject respiratory function is assessed by slow vital capacity (SVC).

5. Zilucoplan for use of any one of claims 1-4, wherein prior to treatment, subject muscle strength is decreased and/or is declining at a specific rate; and wherein treatment with zilucoplan stabilizes or improves subject muscle strength and/or reduces the specific rate at which subject muscle strength is declining, wherein, optionally, change in subject muscle strength is measured by hand held dynamometry (HHD).

6. Zilucoplan for use of any of claims 1-5, wherein zilucoplan treatment stabilizes or improves subject speech capability; and/or stabilizes or improves a rate of subject speech capability decline, wherein, optionally, the subject speech capability comprises one or more of articulatory precision, speaking rate, percent of speech signal voiced, phonation length, consonant length, and nasality.

7. Zilucoplan for use of any of claims 1-6, wherein zilucoplan treatment stabilizes or improves subject ALS biomarker profile; and/or stabilizes or improves a rate of subject ALS biomarker profile change.

8. Zilucoplan for use of claim 7, wherein the subject ALS biomarker profile comprises a biomarker selected from one or more of neurofilament heavy chain (pNfH), neurofilament light chain (NfL), C5, and a complement activity biomarker; wherein, optionally:
(i) the complement activity biomarker is selected from one or more of C5a, C5b-9, soluble C5b-9, C3a, C4a, Ba, Bb, Factor I, and Factor H; and/or
(ii) the complement activity biomarker is detected in subject cerebrospinal fluid (CSF), plasma, and/or serum.

9. Zilucoplan for use of any of claims 1-8, wherein the subject has or is suspected of having myasthenia gravis.

10. Zilucoplan for use of any one of claims 1-9, wherein the subject is positive for at least one autoantibody, wherein, optionally, the at least one autoantibody comprises one or more of anti-acetylcholine receptor antibody, anti-LDL receptor related protein 4, anti-agrin, and anti-muscle associated receptor tyrosine kinase.

11. Zilucoplan for use of any one of claims 1-10, wherein the subject has impaired blood brain barrier function, wherein, optionally, the impaired blood brain barrier function is assessed by determining the subject's albumin quotient.

12. Zilucoplan for use of any one of claims 1-11, wherein the subject is determined to have definite ALS or probable ALS based on revised El Escorial criteria.

13. Zilucoplan for use of any one of claims 1-12, wherein the dose and/or regimen of zilucoplan administered is determined based on the level of a complement activity biomarker detected in CSF, plasma, and/or serum obtained from the subject prior to treatment;
wherein, optionally, the complement activity biomarker detected in CSF, plasma, and/or serum obtained from the subject prior to treatment is selected from one or more of C5a, C5b-9, soluble C5b-9, C3a, C4a, Ba, Bb, Factor I, and Factor H.

14. Zilucoplan for use of any one of claims 1-13, wherein the dose and/or regimen of zilucoplan administered is adjusted after one or more prior zilucoplan administration based on the level of a complement activity biomarker detected in CSF, plasma, and/or serum obtained from the subject after one or more prior administration, wherein, optionally, the complement activity biomarker detected in CSF, plasma, and/or serum obtained from the subject after one or more of prior administration is selected from one or more of C5a, C5b-9, soluble C5b-9, C3a, C4a, Ba, Bb, Factor I, and Factor H.

15. Zilucoplan for use of any one of claims 1-14, wherein the zilucoplan is formulated as a pharmaceutical composition.

## Patentansprüche

1. Zilucoplan zur Verwendung in einem Verfahren zur Behandlung amyotropher Lateralsklerose (ALS) in einem Subjekt, wobei das Verfahren die Verabreichung von Zilucoplan an das Subjekt umfasst.

2. Zilucoplan zur Verwendung gemäß Anspruch 1, wobei vor der Behandlung der ALS-Krankheitsschweregrad des Subjekts erhöht ist und/oder mit einer spezifischen Rate zunimmt; und wobei die Behandlung mit Zilucoplan den ALS-Krankheitsschweregrad des Subjekts stabilisiert oder verringert und/oder die spezifische Rate, mit der der ALS-Krankheitsschweregrad des Subjekts zunimmt, verringert.

3. Zilucoplan zur Verwendung gemäß Anspruch 2, wobei die Veränderung des ALS-Krankheitsschweregrades des Subjekts durch die ALS-Funktionsbewertungsskala - revidiert ("ALS Functional Rating Scale-Revised", ALSFRS-R) gemessen wird.

4. Zilucoplan zur Verwendung gemäß irgendeinem der Ansprüche 1-3, wobei vor der Behandlung die Atemfunktion des Subjekts vermindert ist und/oder mit einer spezifischen Rate abnimmt; und wobei die Behandlung mit Zilucoplant die Atemfunktion des Subjekts stabilisiert oder verbessert und/oder die spezifische Rate, mit der die Atemfunktion des Subjekts abnimmt, verringert, wobei optional die Veränderung der Atemfunktion des Subjekts durch die langsame Vitalkapazität ("slow vital capacity ", SVC) bewertet wird.

5. Zilucoplan zur Verwendung gemäß irgendeinem der Ansprüche 1-4, wobei vor der Behandlung die Muskelkraft des Subjekts vermindert ist und/oder mit einer spezifischen Rate abnimmt; und wobei die Behandlung mit Zilucoplan die Muskelkraft des Subjekts stabilisiert oder verbessert und/oder die spezifische Rate, mit der die Muskelkraft des Subjekts abnimmt, verringert, wobei gegebenenfalls die Veränderung der Muskelkraft des Subjekts durch Hand-Dynamometrie ("hand held dynamometry", HHD) gemessen wird.

6. Zilucoplan zur Verwendung gemäß irgendeinem der Ansprüche 1-5, wobei die Zilucoplan-Behandlung die Sprachfähigkeit des Subjekts stabilisiert oder verbessert; und/oder eine Rate der Abnahme der Sprachfähigkeit des Subjekts stabilisiert oder verbessert, wobei optional die Sprachfähigkeit des Subjekts eines oder mehrere der folgenden Merkmale umfasst: Artikulationspräzision, Sprechgeschwindigkeit, Prozentsatz des stimmhaften Sprachsignals, Phonationslänge, Konsonantenlänge und Nasalität.

7. Zilucoplan zur Verwendung gemäß irgendeinem der Ansprüche 1 bis 6, wobei die Zilucoplan-Behandlung das ALS-Biomarkerprofil des Subjekts stabilisiert oder verbessert; und/oder eine Rate der Änderung des ALS-Biomarkerprofils des Subjekts stabilisiert oder verbessert.

8. Zilucoplan zur Verwendung gemäß Anspruch 7, wobei das ALS-Biomarkerprofil des Subjekts einen Biomarker umfasst, ausgewählt aus einem oder mehr von schwerer Neurofilament-Kette (pNfH), leichter Neurofilament-Kette (NfL), C5 und einem Komplement-Aktivitäts-Biomarker; wobei optional:
(i) der Komplementaktivitäts-Biomarker ausgewählt ist aus einem oder mehreren von C5a, C5b-9, löslichem C5b-9, C3a, C4a, Ba, Bb, Faktor 1 und Faktor H; und/oder
(ii) der Komplementaktivitäts-Biomarker in der zerebrospinalen Flüssigkeit ("cerebrospinal fluid", CSF), im Plasma und/oder im Serum des Subjekts detektiert wird.

9. Zilucoplan zur Verwendung gemäß irgendeinem der Ansprüche 1-8, wobei das Subjekt Myasthenia gravis hat oder der Verdacht besteht, dass es Myasthenia gravis hat.

10. Zilucoplan zur Verwendung gemäß irgendeinem der Ansprüche 1 bis 9, wobei das Subjekt positiv für mindestens einen Autoantikörper ist, wobei optional der mindestens eine Autoantikörper einen oder mehrere von Anti-Acetylcholinrezeptor-Antikörper, Anti-LDL-Rezeptor-verwandtes Protein 4 ("anti-LDL receptor related protein 4"), Anti-Agrin und Anti-Muskel-assoziierte Rezeptor-Tyrosinkinase ("antimuscle associated receptor tyrosine kinase") umfasst.

11. Zilucoplan zur Verwendung gemäß irgendeinem der Ansprüche 1-10, wobei das Subjekt eine gestörte Blut-Hirn-Schrankenfunktion hat, wobei optional die gestörte Blut-Hirn-Schrankenfunktion durch Bestimmen des Albuminquotienten des Subjekts beurteilt wird.

12. Zilucoplan zur Verwendung gemäß irgendeinem der Ansprüche 1-11, wobei bestimmt wird, dass das Subjekt definitiv ALS oder wahrscheinliche ALS hat, basierend auf den revidierten El Escorial-Kriterien ("revised El Escorial criteria").

13. Zilucoplan zur Verwendung gemäß irgendeinem der Ansprüche 1-12, wobei die Dosis und/oder das Regime von verabreichtem Zilucoplan basierend auf dem Level eines Komplementaktivitäts-Biomarkers bestimmt wird, der in CSF, Plasma und/oder Serum, das von dem Subjekt vor der Behandlung erhalten wurde, nachgewiesen wurde;
wobei optional der Komplementaktivitäts-Biomarker, der in CSF, Plasma und/oder Serum, das von dem Subjekt vor der Behandlung erhalten wurde, nachgewiesen wurde, ausgewählt ist aus einem oder mehreren von C5a, C5b-9, löslichem C5b-9, C3a, C4a, Ba, Bb, Faktor 1 und Faktor H.

14. Zilucoplan zur Verwendung gemäß irgendeinem der Ansprüche 1-13, wobei die verabreichte Dosis und/oder das Regime von Zilucoplan nach einer oder mehreren vorherigen Zilucoplan-Verabreichungen basierend auf dem Level eines Komplementaktivitäts-Biomarkers, der in CSF, Plasma und/oder Serum, das von dem Subjekt nach einer oder mehreren vorherigen Verabreichungen erhalten wurde, nachgewiesen wurde, angepasst wird, wobei optional der Komplementaktivitäts-Biomarker, der in CSF, Plasma und/oder Serum, das von dem Subjekt nach einer oder mehreren früheren Verabreichungen erhalten wurde, nachgewiesen wurde, aus einem oder mehreren von C5a, C5b-9, löslichem C5b-9, C3a, C4a, Ba, Bb, Faktor 1 und Faktor H ausgewählt ist.

15. Zilucoplan zur Verwendung gemäß irgendeinem der Ansprüche 1-14, wobei das Zilucoplan als eine pharmazeutische Zusammensetzung formuliert ist.

## Revendications

1. Zilucoplan pour une utilisation dans un procédé de traitement de la sclérose latérale amyotrophique (SLA) chez un sujet, le procédé comprenant l'administration de zilucoplan au sujet.

2. Zilucoplan pour une utilisation selon la revendication 1, dans lequel, avant le traitement du sujet, la sévérité de la maladie de la SLA est élevée et/ou augmente à une vitesse spécifique ; et
dans lequel le traitement par zilucoplan stabilise ou réduit la sévérité de la maladie de la SLA du sujet, et/ou réduit la vitesse spécifique à laquelle la sévérité de la maladie de SLA du sujet augmente.

3. Zilucoplan pour une utilisation selon la revendication 2, dans lequel la variation de la sévérité de la maladie de la SLA chez le sujet est mesurée par l'échelle fonctionnelle de la SLA révisée (ALSFRS-R).

4. Zilucoplan pour une utilisation selon l'une quelconque des revendications 1 à 3, dans lequel, avant le traitement, la fonction respiratoire du sujet est diminuée et/ou décline à une vitesse spécifique ; et dans lequel le traitement par zilucoplan stabilise ou améliore la fonction respiratoire du sujet et/ou réduit la vitesse spécifique à laquelle la fonction respiratoire du sujet diminue, dans lequel, facultativement, la modification de la fonction respiratoire du sujet est évaluée par la capacité vitale lente (CVL).

5. Zilucoplan pour une utilisation selon l'une quelconque des revendications 1 à 4, dans lequel, avant le traitement, la force musculaire du sujet est diminuée et/ou décline à une vitesse spécifique ; et dans lequel le traitement par zilucoplan stabilise ou améliore la force musculaire du sujet et/ou réduit la vitesse spécifique à laquelle la force musculaire du sujet diminue, dans lequel, facultativement, la modification de la force musculaire du sujet est mesurée par dynamométrie manuelle (HHD).

6. Zilucoplan pour une utilisation selon l'une quelconque des revendications 1 à 5, dans lequel le traitement par zilucoplan stabilise ou améliore la capacité vocale du sujet ; et/ou stabilise ou améliore une vitesse de déclin de la capacité vocale du sujet, dans lequel, facultativement, la capacité vocale du sujet comprend une ou plusieurs précisions articulaires, vitesse de parole, pourcentage du signal de parole voisé, longueur de phonation, longueur de consonne et nasalité.

7. Zilucoplan pour une utilisation selon l'une quelconque des revendications 1 à 6, dans lequel le traitement par zilucoplan stabilise ou améliore le profil de biomarqueur de la SLA du sujet ; et/ou stabilise ou améliore une vitesse de changement du profil de biomarqueur de la SLA du sujet.

8. Zilucoplan pour une utilisation selon la revendication 7, dans lequel le profil de biomarqueur de la SLA du sujet comprend un biomarqueur sélectionné parmi une ou plusieurs chaînes lourdes de neurofilaments (pNfH), chaînes légères de neurofilaments (NfL), C5 et un biomarqueur d'activité de complément ; dans lequel, éventuellement :
(i) le biomarqueur d'activité de complément est sélectionné parmi un ou plusieurs de C5a, C5b-9, C5b-9 soluble, C3a, C4a, Ba, Bb, facteur I, et facteur H ; et/ou
(ii) le biomarqueur d'activité de complément est détecté dans le liquide céphalorachidien (LCR), le plasma et/ou le sérum du sujet.

9. Zilucoplan pour une utilisation selon l'une quelconque des revendications 1 à 8, dans lequel le sujet présente ou est suspecté de présenter une myasthénie grave.

10. Zilucoplan pour une utilisation selon l'une quelconque des revendications 1 à 9, dans lequel le sujet est positif pour au moins un auto-anticorps, dans lequel, facultativement, l'au moins un auto-anticorps comprend un ou plusieurs parmi l'anticorps anti-récepteur de l'acétylcholine, la protéine 4 liée au récepteur anti-LDL, l'anti-agrine et l'anti-récepteur tyrosine kinase associé au muscle.

11. Zilucoplan pour une utilisation selon l'une quelconque des revendications 1 à 10, dans lequel le sujet présente une fonction de barrière hématoencéphalique altérée, dans lequel, facultativement, la fonction de barrière hématoencéphalique altérée est évaluée en déterminant le quotient d'albumine du sujet.

12. Zilucoplan pour une utilisation selon l'une quelconque des revendications 1 à 11, dans lequel le sujet est déterminé à présenter une SLA définitive ou probable sur la base des critères révisés d'El Escorial.

13. Zilucoplan pour une utilisation selon l'une quelconque des revendications 1 à 12, dans lequel la dose et/ou le régime de zilucoplan administré(e) est déterminé(e) sur la base du niveau d'un biomarqueur d'activité de complément détecté dans le LCR, le plasma et/ou le sérum obtenu auprès du sujet avant le traitement ; dans lequel, éventuellement, le biomarqueur d'activité de complément détecté dans le LCR, le plasma et/ou le sérum obtenu auprès du sujet avant le traitement est sélectionné parmi un ou plusieurs des C5a, C5b-9, C5b-9 soluble, C3a, C4a, Ba, Bb, facteur I et facteur H.

14. Zilucoplan pour une utilisation selon l'une quelconque des revendications 1 à 13, dans lequel la dose et/ou le régime de zilucoplan administré(e) est ajusté(e) après une ou plusieurs administrations antérieures de zilucoplan sur la base du niveau d'un biomarqueur d'activité de complément détecté dans le LCR, le plasma et/ou le sérum obtenu chez le sujet après une ou plusieurs administrations antérieures, dans lequel, éventuellement, le biomarqueur d'activité de complément détecté dans le LCR, le plasma et/ou le sérum obtenu auprès du sujet après une ou plusieurs administrations antérieures est sélectionné parmi un ou plusieurs des C5a, C5b-9, C5b-9 soluble, C3a, C4a, Ba, Bb, facteur I et facteur H.

15. Zilucoplan pour une utilisation selon l'une quelconque des revendications 1 à 14, dans lequel le zilucoplan est formulé en tant que composition pharmaceutique.
